(19) Europäisches Patentamt
European Patent Office
Office européen des brevets

(11) Publication number: **0 494 283 B1**

(12) # EUROPEAN PATENT SPECIFICATION

(45) Date of publication of patent specification: **11.10.95**

(51) Int. Cl.[6]: **C07D 405/12**, C07D 319/20

(21) Application number: **91912540.1**

(22) Date of filing: **10.07.91**

(86) International application number:
**PCT/EP91/01289**

(87) International publication number:
**WO 92/02517 (20.02.92 92/05)**

(54) **1,4-BENZODIOXANE DERIVATIVES, PROCESS FOR THE PREPARATION THEREOF AND PHARMACEUTICAL COMPOSITIONS THEREFROM.**

(30) Priority: **27.07.90 ES 9002029**

(43) Date of publication of application:
**15.07.92 Bulletin 92/29**

(45) Publication of the grant of the patent:
**11.10.95 Bulletin 95/41**

(84) Designated Contracting States:
**AT BE CH DE DK ES FR GB GR IT LI LU NL SE**

(56) References cited:
**FR-A- 2 358 142**

(73) Proprietor: **LABORATORIOS MENARINI S.A.**
**Alfonso XII, 587**
**E-08912 Badalona (ES)**

(72) Inventor: **MAULEON, Casellas**
**Alfonso XII, 587**
**E-08912 Badalona (ES)**
Inventor: **CARGANICO, Germano**
**Alfonso XII, 587**
**E-08912 Badalona (ES)**
Inventor: **LOBATO, Rodriguez**
**Alfonso XII, 587**
**E-08912 Badalona (ES)**

(74) Representative: **Minoja, Fabrizio**
**Studio Consulenza Brevettuale,**
**Via Rossini, 8**
**I-20122 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to novel 1,4-benzodioxane derivatives having antitumor and antiplatelet aggregating activities, to a process for the preparation thereof and to pharmaceutical compositions containing them.

## TECHNOLOGICAL BACKGROUND

Platelet activating factor (PAF), or 1-O-alkyl-2-acetyl-sn-glyceryl-3-phosphorylcholine, of formula A

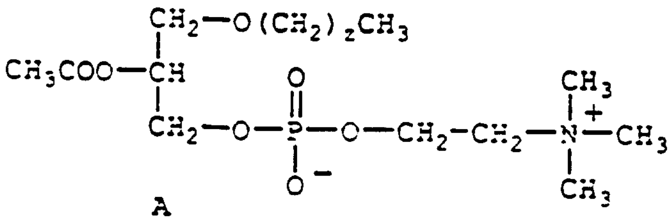

A

wherein z is 15 or 17,
is a potent cell mediator of phospholipid nature, which is ascribed to play a paramount role in various pathological processes, such as asthma, anaphylaxis, septic shock, transplant rejection, inflammation, ulcerogenesis and ischemia [P. Braquet et al., Pharmacol. Rev., 39(2), 97 (1987)].

Various searches evidenced the existence of a specific PAF-receptor in various human cells, as well as the capability of different compounds to antagonize PAF action at the level of said receptor [K. Cooper and M.J. Parry, Ann. Rep. Med. Chem., 24, 81 (1989)].

Therefore, specific PAF-antagonists can be interesting in therapy for the treatment of those pathological processes in which PAF is directly or indirectly involved.

Another class of synthetic phospholipids is represented by those named alkyl-lyso-phospholipids (ALPs), which are structurally related to PAF and lyso-phosphatidylcholine; for example, ET-18-$OCH_3$, of formula B

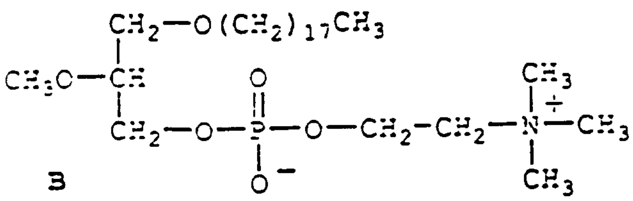

B

Said compounds show a marked antitumor activity in various pharmacological models [W.E. Berdel and P.G. Munder in "PAF and related lipid mediators", Ed. F. Snyder, Plenum Press, New York, 1987, p. 449]. Undoubtedly, ET-18-$OCH_3$ and other analogues, due to the structural relationship thereof with PAF, have some of PAF actions, such as serotonine release, bronchoconstriction, platelet aggregation and hypotension. Said actions are contra-indicated in case of antitumor treatment and they can cause remarkable circulatory disorders.

As a consequence, a compound having the cytostatic action of those phospholipids which are related with compound B, and lacking the PAF-like action or, even better, capable to block PAF action on the specific cell receptors thereof, could advantageously be used in human therapy as an antitumor agent, without suffering from the drawbacks of compound B. Moreover, the PAF-antagonistic properties will allow the use of such a compound in any disorder related to said mediator.

## DISCLOSURE OF THE INVENTION

The present invention relates to novel 1,4-benzodioxane derivatives and pharmaceutically acceptable salts thereof, as well as to a process for the preparation thereof and to pharmaceutical compositions containing them.

Specifically, the present invention relates to compounds of formula (I)

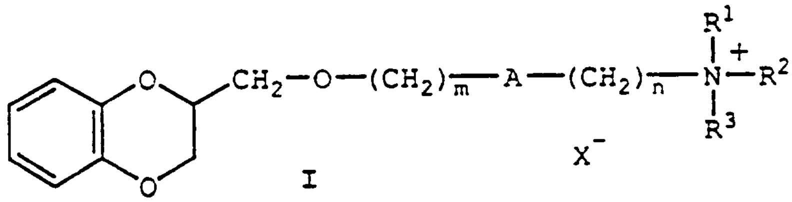

I

wherein $R^1$, $R^2$ and $R^3$, which can be the same or different, are hydrogen, lower alkyl, aryl or lower aralkyl groups;

or $R^1R^2R^3N^+$ is an aromatic cyclic ammonium group, such as 1-pyridinium, 1-quinolinium, 2-isoquinolinium, 1-imidazolium, 1-pyrazolium, 3-thiazolium, 3-oxazolium, 1-benzimidazolium, 3-benzothiazolium or 3-benzoxazolium, having on its ring from 1 to 3 substituents, such as lower alkyl, halogen, lower hydroxyalkyl, amino, lower aminoalkyl or carboxy;

or $R^1R^2R^3N^+$ is a non aromatic cyclic ammonium group, in which two of groups ($R^1$, $R^2$ or $R^3$), together with the nitrogen atom, form a ring such as 1-pyrrolidine, 1-piperidine, 4-morpholine, 1-piperazine or 1-perhydroazepine and the remaining group is hydrogen or lower alkyl;

m and n, which can be the same or different, are an integer from 2 to 12;

A is either a single covalent bond (i.e., it represents no groups), or it is an oxygen atom;

$X^-$ is a pharmaceutically acceptable anion from an inorganic acid (hydrochloric, hydrobromic, hydroiodic or sulfuric acid) or from a carboxylic organic acid (such as acetic, lactic, oxalic, tartaric, citric or malic acid), or from an organic sulfonic acid (such as methanesulfonic, ethanesulfonic, benzenesulfonic or toluenesulfonic acid).

The present invention also relates to the stereoisomers of compounds (I) and the mixtures thereof.

As herein used, "lower alkyl" means straight or branched $C_1$-$C_6$ alkyl groups; "aralkyl" means straight or branched $C_1$-$C_6$ alkyl groups substituted with an aromatic ring, preferably a phenyl ring.

The compounds of the present invention are a novel structural class, since the compounds of formula (I) having a long side-chain, with $m + n \geqq 4$, have not hitherto been disclosed.

Moreover, compounds (I) have marked cytotoxic and/or PAF-antagonist activities. In fact, no 1,4-benzodioxane derivative has hitherto been found to have such pharmacological activities.

Preferred compounds of the invention are those in which:

- m + n = 7-22;
- $R^1$, $R^2$ and $R^3$ are hydrogen, lower alkyl, aryl or lower aralkyl groups, preferably methyl or ethyl;
- $R^1R^2R^3N^+$ is an aromatic cyclic ammonium group as defined above, preferably 1-pyridinium, 1-imidazolium or 3-thiazolium;
- $R^1R^2R^3N^+$ is a non aromatic cyclic ammonium group as defined above, preferably a 1-pyrrolidinium, 1-piperidinium, 4-morpholinium, 1-methyl-1-pyrrolinium, 1-methyl-1-piperidinium or 4-methyl-4-morpholinium group.

Particularly preferred compounds are the following ones:

N-[10-[[(1,4-benzodioxan-2-yl)methyl]oxy]decyl]-N,N,N-trimethylammonium,
N-[16-[[(1,4-benzodioxan-2-yl)methyl]oxy]hexadecyl]-N,N,N-trimethylammonium,
N-[6-[[10-[[(1,4-benzodioxan-2-yl)methyl]oxy]decyl]oxy]hexyl]-N,N,N-trimethylammonium,
N-[10-[[10-[[(1,4-benzodioxan-2-yl)methyl]oxy]decyl]oxy]decyl]-N,N,N-trimethylammonium,
N-[10-[[(1,4-benzodioxan-2-yl)methyl]oxy]decyl]-N,N,N-triethylammonium,
N-[16-[[(1,4-benzodioxan-2-yl)methyl]oxy]hexadecyl]-N,N,N-triethylammonium,
N-[10-[[(1,4-benzodioxan-2-yl)methyl]oxy]decyl]-N-methylpiperidinium,
N-[16-[[(1,4-benzodioxan-2-yl)methyl]oxy]hexadecyl]-N-methylpiperidinium,
N-[6-[[10-[[(1,4-benzodioxan-2-yl)methyl]oxy]decyl]oxy]hexyl]-N-methylpiperidinium,
N-[10-[[10-[[(1,4-benzodioxan-2-yl)methyl]oxy]decyl]oxy]decyl]-N-methylpiperidinium,
N-[10-[[(1,4-benzodioxan-2-yl)methyl]oxy]decyl]-N-methylmorpholinium,
N-[10-[[(1,4-benzodioxan-2-yl)methyl]oxy]decyl]piperidinium,
N-[16-[[(1,4-benzodioxan-2-yl)methyl]oxy]hexadecyl]piperidinium,
N-[10-[[6-[[(1,4-benzodioxan-2-yl)methyl]oxy]decyl]oxy]hexyl]piperidinium,
N-[10-[[10-[[(1,4-benzodioxan-2-yl)methyl]oxy]decyl]oxy]decyl]piperidinium,
N-[10-[[(1,4-benzodioxan-2-yl)methyl]oxy]decyl]morpholinium,
(2S)-N-[10-[[(1,4-benzodioxan-2-yl)methyl]oxy]decyl]piperidinium,

(2R)-N-[10-[[(1,4-benzodioxan-2-yl)methyl]oxy]decyl]piperidinium, wherein $X^-$ is as defined above.

According to the present invention, compounds (I) can be prepared by reacting a compound of formula (II)

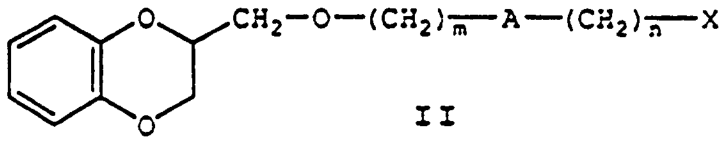

II

wherein X is an appropriate leaving group, for example halogen (such as chlorine, bromine or iodine), lower alkylsulfonate (such as methanesulfonate), arylsulfonate (such as benzenesulfonate or toluenesulfonate), with a nitrogen base of formula $NR^1R^2R^3$, in a suited solvent, which can be selected either from aprotic dipolar solvents, such as acetonitrile or dimethylformamide; aromatic hydrocarbons, such as benzene, toluene or xylene; halogenated aliphatic hydrocarbons, such as chloroform or dichloromethane, or ethers, such as ethyl ether, tetrahydrofuran or dioxane; or it can be the nitrogen base $NR^1R^2R^3$ itself. The reaction is carried out at a temperature ranging from 15 °C to the solvent's reflux temperature, for a time ranging from 1 to 48 hours. Thereafter, the resulting compound (I) can optionally be salified.

Starting compound (II) can be prepared according to the following Reaction Scheme 1.

**Scheme 1**

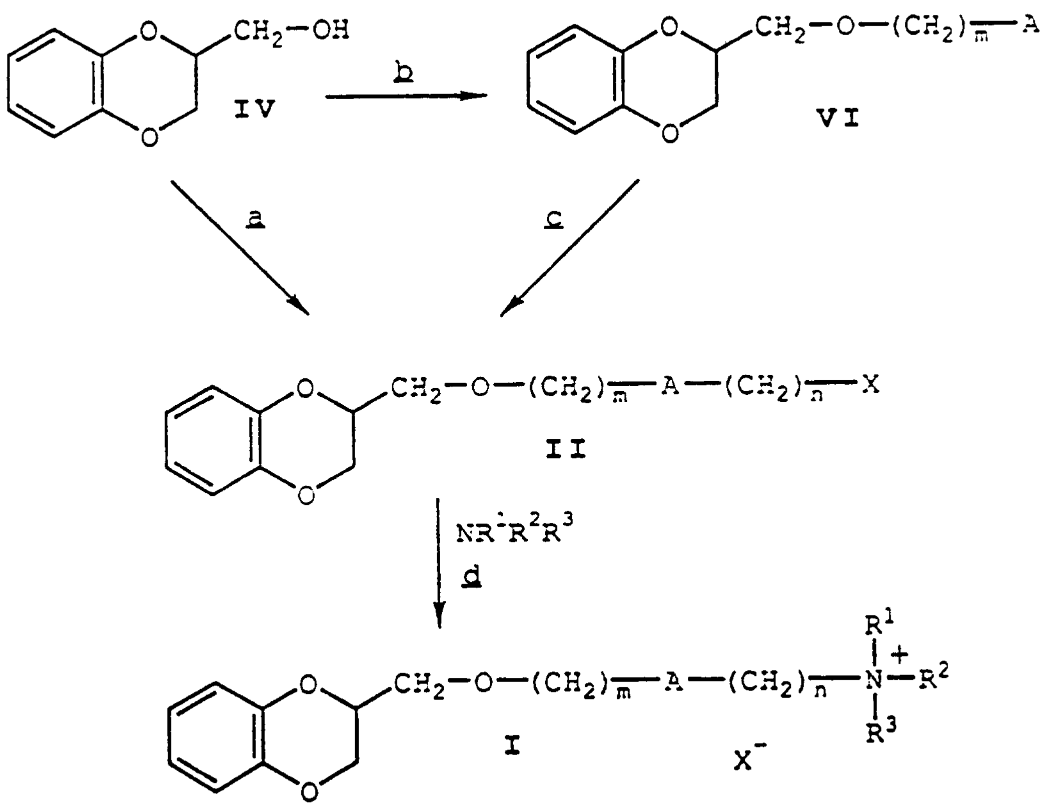


Both compound (IV) and the preparation thereof in the racemic and enantiomerically pure forms are described in literature [A. Delgado, G. Leclerc, M.C. Lobato and D. Mauleon, Tetrahedron Lett. 29, 3671 (1988), and references cited therein].

According to step a of Scheme 1, a compound of formula (II) can be obtained, for example, by reacting 1,4-benzodioxane-2-ylmethanol (IV) with a compound (V) of formula $Y-(CH_2)_m-A-(CH_2)_n-X$, wherein Y is an appropriate leaving group (for example bromide, iodide, mesylate or tosylate) and X has the above

mentioned meanings or (especially when m and n are different from each other), it is a suitably protected alcohol, for example by means of a benzyl group. Compounds of formula (V) are commercially available and, if not, they can easily be prepared starting from the corresponding alcohols HO-$(CH_2)_m$-A-$(CH_2)_n$-OH. Thus, compounds (V) in which X is benzyloxy and Y is bromine can be prepared, for example, starting from the corresponding 1,$\omega$-diol by mono-benzylation (1 benzyl bromide equivalent, 1 NaH equivalent), followed by substitution of the remaining OH by bromine (by means of phosphorous tribromide, phosphorus pentabromide or the reagent consisting of triphenyl phosphin and carbon tetrabromide, all of them in an inert solvent). After that, step a comprises reacting the compound of formula (IV) first with a strong base, such as sodium hydride or potassium hydride, then with compound (V) in a dipolar aprotic solvent, such as dimethylformamide or dimethyl sulfoxide. The reaction is carried out at a temperature ranging from 15 to 100°C, for a time between 1 and 24 hours. When X is a O-protecting group, this step is followed by deprotection to the corresponding alcohol (for example by hydrogenation on palladium catalyst, when the protecting group is a benzyl group) and by transformation of said alcohol into a second leaving group X (such as bromide, tosylate or any one of the above mentioned ones).

According to steps b and c of Scheme 1, a compound of formula (II), in which A is oxygen, X , m and n have the above mentioned meanings, can also be obtained in two steps by reacting benzodioxanyl-methanol (IV) with a compound of formula Z-$(CH_2)_m$-OP, wherein Z is an appropriate leaving group (such as an halide or an alcohol sulfonate) and P is an oxygen-protecting group (such as benzyl, silyl ether or tetrahydropyranyl), followed by deprotection of the alcohol and by a second alkylation with a compound Y-$(CH_2)_n$-X, wherein Y, X and n have the above mentioned meanings. More particularly, the alkylation reaction of (IV) (step b) to give intermediate compound (VI) is carried out under similar conditions to those for step a, with the same kind of reagents and solvents. Compound (VI) can easily be deprotected according to well-known methods (for example, by hydrogenolysis when P is a benzyl group or by hydrolysis with hydrochloric acid when R is a tetrahydropyranyl group), thereby obtaining an alcohol (VI) (A-P = OH). Step c consists in reacting the resulting alcohol with a compound $Y(CH_2)_nX$, using the same reagents and conditions as those used in step a to obtain compound (II).

A compound of formula (II) in which A is a single bond, X, m and n have the above mentioned meanings, can also be obtained starting from a compound (VI), wherein A-P = Br, preparing a Grignard reagent by treatment with metal magnesium, then adding said reagent to a compound of formula $Y(CH_2)_nX$ (wherein Y, X and n have the above mentioned meanings), said addition being catalysed by copper compounds (such as lithium tetrachlorocuprate).

Finally, step d of Scheme 1 gives compounds of formula (I), by reacting a compound of formula (II) with a nitrogen base (III) of formula $NR^1R^2R^3$. Step d is conveniently carried out in an inert organic solvent, such as benzene, toluene, chloroform, acetonitrile, dimethylformamide, ethanol or methanol, at a temperature from 15°C to the reflux temperature of the solvent. Where the nitrogen base has a boiling point below 30°C (for example when $R^1 = R^2 = R^3 = CH_3$), the reaction can be effected in a sealed tube having thick walls. Once compound (I) has been obtained, the $X^-$ anion can be replaced by another anion, for example by chromatography on a ion exchange resin.

Alternatively, a compound of formula (I), wherein at least one of the $R^1$, $R^2$ or $R^3$ groups is a lower alkyl group, can also be obtained starting from a compound of formula (I) in which at least one of the $R^1$, $R^2$ or $R^3$ groups is hydrogen, by treatment with an inorganic base, such as sodium hydroxide, to obtain the free nitrogen base, followed by reaction with a compound of formula XR, wherein X has the above mentioned meanings and R is a lower alkyl group. Examples of reactives XR are methyl iodide, methyl bromide or ethyl bromide. This reaction is conveniently carried out in an inert organic solvent such as toluene, benzene, chloroform, acetonitrile, dimethylformamide, ethanol or methanol, at a temperature from 0°C to the solvent reflux temperature.

As already stated, compounds (I) of the present invention inhibit PAF-induced platelet aggregation, thus they are valuable as PAF-antagonists in the treatment of those diseases this substance is involved with. Further, compounds (I) have also a marked antitumor activity, which has been determined, for example, as the cytotoxic activity against tumor cells. Therefore compounds (I) can be used as useful antitumor agents. Contrarily to PAF, benzodioxanes of the present invention show no negative effects related to this mediator, which promotes the therapeutical use thereof, since they show a remarkable decrease in undesired side-effects.

The compounds of the invention can be used in human therapy as antitumor, antiasthmatic, antithrombotic, anti-inflammatory, anti-allergic agents as well as in prophylaxis of anaphylactic shock. Moreover, they can be used as hypotensive or antianginal agents.

For this purpose, the compounds of the invention can be formulated according to conventional techniques and excipients, such as those described in "Remington's Pharmaceutical Science Handbook",

Mack Pub. Co., New York, U.S.A. Examples of said forms include capsules, tablets, syrups and the like, containing 1 to 1000 mg of the active ingredient per unitary dose.

The following Examples further illustrate the invention.

## EXAMPLE 1

**N-[4-[[(1,4-Benzodioxan-2-yl)methyl]oxy]butyl]-N,N,N-trimethylammonium bromide (I, $R^1$ = $R^2$ = $R^3$ = $CH_3$, A = single bond, X = Br, m = n = 2).**

a) 2-[[(4-Bromobutyl)oxy]methyl]-1,4-benzodioxane (II, A = single bond, X = Br, m = n = 2).

A suspension of sodium hydride (50%, 7.20 g, 0.15 mole) in 100 ml of anhydrous dimethylformamide is prepared, which is then added first with a solution of 1,4-dibromobutane (28.08 g, 0.13 mole) in 25 ml of anhydrous dimethylformamide, then with a solution of 2-hydroxymethyl-1,4-benzodioxane (IV, 8.30 g, 0.05 mole) in 25 ml of anhydrous dimethylformamide, dropwise. The reaction mixture is stirred at 60 °C for 18 hours, thereafter ethanol and water (400 ml) are added. The mixture is extracted with ethyl ether (4 x 50 ml), the ether phases are washed with a saturated sodium chloride solution, dried over anhydrous sodium sulfate and evaporated to dryness. The resulting residue is distilled under reduced pressure (0.5 - 2 Torr), thus removing the 1,4-dibromobutane excess (b.p. 40 °C, bath temperature). Bromo-derivative 1a, distilling at 90-110 °C, is obtained in a 78% yield .

$^1H$ NMR (200 MHz, $CDCl_3$) $\delta$(TMS): 1.82 and 2.10 (m, 4H, central $CH_2$ of the chain), 3.45 - 3.80 (m, 6H, $CH_2OCH_2$ + $CH_2Br$), 4.12 (dd, 1H, benzodioxane $C^2H$), 4.36 (m, 2H, benzodioxane $C^3H_2$), 6.90 (m, 4H, benzodioxane $C^5H$-$C^8H$).

b) Preparation of the title compound.

A solution of 4 g of dry trimethylamine in 20 ml of anhydrous acetonitrile is prepared in a sealed reaction tube, keeping it cold with a solid $CO_2$ and acetone outer bath. 3.01 g (0.01 mole) of bromo-derivative 1a are added to this solution, the reactor is sealed and immersed in an oil bath at 60-70 °C. After 18 hours the reaction mixture is cooled, solvent is evaporated to dryness and the residue is crystallized twice from anhydrous acetone-anhydrous ethyl ether, to obtain the title ammonium salt in an 81% yield, m.p. 142-143 °C. The most significant NMR data are reported in Tables I and II.

**TABLE I. $^1$H NMR spectra of the example compounds.**

| Ex. Nº | Signal assignments:* 1 | 2 | 3 | 4 | 5 | 6 |
|---|---|---|---|---|---|---|
| 1 | 1,53 y 1,80 | 3,34 | 3,50-3,68 | 4,01 | 4,27 | 6,80 |
| 2 | 1,40-1,90 | 3,45 | 3,50-3,75 | 4,07 | 4,30 | 6,88 |
| 3 | 1,30-1,80 | 3,34 | 3,50-3,65 | 3,98 | 4,22 | 6,76 |
| 4 | 1,20-1,85 | 3,45 | 3,40-3,70 | 4,04 | 4,26 | 6,82 |
| 5 | 1,22-2,10 | - | 3,50-3,80 | 4,12 | 4,30 | 6,91 |
| 8 | 1,45 y 1,59 | 1,04 | 3,00-3,12<br>3,35-3,50 | 4,03 | 4,27 | 6,82 |
| 9 | 1,30-1,70 | 1,20 | 3,18-3,35<br>3,45-3,60 | 3,96 | 4,24 | 6,79 |
| 10 | 1,16-1,68 | 1,25 | 3,12-3,45<br>3,50-3,65 | 4,00 | 4,23 | 6,80 |
| 11 | 1,22-2,10 | 1,18 | 3,48-3,75 | 4,07 | 4,30 | 6,85 |
| 12 | 1,50-1,60 | - | 2,20-2,35<br>3,40-3,70 | 4,03 | 4,28 | 6,81 |
| 13 | 1,40-1,70 | - | 2,25-2,45<br>3,45-3,70 | 4,07 | 4,28 | 6,87 |
| 14 | 1,28-1,70 | - | 2,30-2,45<br>3,45-3,75 | 4,08 | 4,28 | 6,89 |
| 15 | 1,19-1,60 | - | 2,20-2,38<br>3,43-3,68 | 4,03 | 4,37 | 6,81 |
| 16 | 1,68 y 2,02 | - | 2,80-2,55<br>3,50-3,70 | ~4,1 | ~4,3 | 6,85 |
| 17 | 1,30-1,70 | - | 2,35-2,55<br>3,50-3,70 | 4,07 | 4,27 | 6,85 |
| 18 | 1,25-1,68 | - | 2,30-2,50<br>3,45-3,75 | 4,06 | 4,32 | 6,83 |
| 19 | 1,16-1,90 | - | 2,60-2,90<br>3,40-3,70 | 4,05 | 4,25 | 6,82 |

**TABLE I (follows)**

| Ex. | Signal assignments:* | | | | | |
|---|---|---|---|---|---|---|
| Nº | 1 | 2 | 3 | 4 | 5 | 6 |
| 20 | 1,64 y 1,80 | 3,24 | 3,45-3,70 | 4,00 | 4,30 | 6,80 |
| 21 | 1,60-1,90 | 3,27 | 3,45-3,75 | 4,05 | 4,25 | 6,82 |
| 22 | 1,38-1,90 | 3,23 | 3,43-3,75 | 3,98 | 4,23 | 6,80 |
| 23 | 1,20-1,90 | 3,28 | 3,40-3,78 | 4,02 | 4,27 | 6,80 |
| 24 | 1,22-2,10 | 3,39 | 3,52-3,80 | 4,10 | 4,35 | 6,90 |
| 27 | 1,54 y 1,83 | 3,30 | 3,50-3,70 | ~4,0 | 4,30 | 6,81 |
| 28 | 1,48-1,97 | 3,50 | 3,50-3,70 | 4,07 | 4,32 | 6,85 |
| 29 | 1,42-1,92 | 3,52 | 3,50-3,75 | 4,05 | 4,32 | 6,87 |
| 30 | 1,23-1,82 | 3,53 | 3,45-3,82 | 4,05 | 4,30 | 6,82 |
| 31 | 1,58 y 1,85 | 1,25 | 2,90-3,10<br>3,45-3,65 | 3,95 | 4,26 | 6,80 |
| 32 | 1,22-1,65 | 1,00 | 2,35-2,50<br>3,45-3,65 | 4,03 | 4,28 | 6,82 |
| 33 | 0,99-1,58 | - | 2,30-2,51<br>3,41-3,72 | 4,05 | 4,25 | 6,83 |
| 34 | 1,12-1,68 | - | 2,22-2,43<br>3,41-3,75 | 4,04 | 4,35 | 6,86 |

* 1: central $CH_2$ groups of the side chain; 2: N-$CH_3$; 3: N-$CH_2$ and non-benzodioxane O-$CH_2$ groups; 4: benzodioxane $C^2H$; 5: benzodioxane $C^3H_2$; 6: aromatic H in benzodioxane.

**TABLE II. $C^{13}$ NMR spectra of the example compounds.**

| Ex. Nº | Signal assignments:* 1 | 2 | 3 | 4 | 5 | 6 | 7 | 8 | 9 | 10 |
|---|---|---|---|---|---|---|---|---|---|---|
| 1 | 20,3<br>26,2 | 53,5 | 65,8 | 66,7 | 69,9 | 70,8 | 72,5 | 117,7<br>117,8 | 122,0<br>122,2 | 143,7<br>143,8 |
| 2 | 22,9<br>23,0<br>29,0 | 53,4 | 65,7 | 66,8 | 69,6 | 71,4 | 72,3 | 117,5<br>117,7 | 121,7<br>121,9 | 143,5 |
| 3 | 23,2<br>25,8<br>26,0<br>29,3 | 53,5 | 65,8 | 66,8 | 69,6 | 71,7 | 72,3 | 117,5<br>117,7 | 121,7<br>121,9 | 143,5<br>143,6 |
| 4 | 23,4<br>a<br>29,7 | 53,7 | 66,1 | 67,4 | 69,7 | 72,4 | 72,4 | 117,6<br>117,9 | 121,9<br>122,1 | 143,7 |
| 8 | 18,7<br>26,2 | 6,7 | 65,7 | 53,0<br>56,9 | 70,0 | 70,8 | 72,9 | 117,5 | 121,8<br>121,9 | 144,0<br>144,1 |
| 9 | 22,2<br>25,9<br>26,4<br>29,4 | 8,2 | 65,9 | 53,8<br>57,7 | 69,7 | 71,8 | 72,5 | 117,6<br>117,9 | 121,9<br>122,1 | 143,7<br>143,8 |
| 10 | 23,0<br>a<br>29,9 | 8,0 | 65,8 | 66,8 | 68,9 | 70,7 | 72,5 | 117,6<br>117,9 | 121,9<br>122,1 | 143,8 |
| 12 | 24,7<br>27,9 | - | 66,0 | 54,9<br>59,6 | 69,7 | 72,2 | 72,5 | 117,9 | 121,9<br>122,1 | 143,8<br>144,9 |

**TABLE II (follows)**

| Ex. Nº | Signal assignments:* 1 | 2 | 3 | 4 | 5 | 6 | 7 | 8 | 9 | 10 |
|---|---|---|---|---|---|---|---|---|---|---|
| 14 | 26,3 | - | 66,0 | 55,2 | 69,8 | 72,5 | 72,6 | 117,6 | 121,8 | 143,7 |
| | 27,1 | | | 60,0 | | | | 117,9 | 122,0 | |
| | 28,1 | | | | | | | | | |
| | 30,0 | | | | | | | | | |
| 15 | 24,7 | - | 66,1 | 55,0 | 69,7 | 72,4 | 72,4 | 117,6 | 121,8 | 143,8 |
| | a | | | 60,1 | | | | 117,9 | 122,1 | 143,9 |
| | 29,8 | | | | | | | | | |
| 16 | 20,1 | - | 65,1 | 51,4 | 69,3 | 70,4 | 71,8 | 117,0 | 121,3 | 143,0 |
| | 26,2 | | | 57,2 | | | | 117,1 | 121,5 | |
| 18 | 26,6 | - | 66,2 | 54,3 | 69,8 | 72,5 | 72,6 | 117,6 | 121,8 | 143,1 |
| | 27,0 | | | 59,6 | | | | 117,9 | 122,1 | |
| | 27,8 | | | | | | | | | |
| | 30,0 | | | | | | | | | |
| 19 | 24,6 | - | 66,7 | 53,1 | 70,5 | 72,9 | 73,7 | 118,2 | 122,5 | 143,8 |
| | a | | | 58,6 | | | | 118,4 | 122,7 | |
| | 30,6 | | | | | | | | | |
| 20 | 19,2 | 48,5 | 65,8 | 61,2 | 69,9 | 70,9 | 72,5 | 117,7 | 122,0 | 143,7 |
| | 26,4 | | | 62,9 | | | | 117,8 | 122,1 | 143,8 |
| 21 | 22,1 | 48,9 | 65,9 | 61,5 | 69,9 | 71,7 | 72,5 | 117,7 | 121,9 | 143,8 |
| | 23,4 | | | 63,6 | | | | 117,9 | 122,2 | |
| | 29,2 | | | | | | | | | |
| 22 | 22,2 | 48,8 | 66,0 | 61,4 | 69,8 | 71,9 | 72,5 | 117,6 | 121,9 | 143,8 |
| | 25,9 | | | 63,7 | | | | 117,9 | 122,1 | |
| | 26,2 | | | | | | | | | |
| | 29,4 | | | | | | | | | |
| 23 | 20,9 | 48,8 | 66,1 | 61,2 | 69,7 | 72,4 | 72,4 | 117,6 | 121,9 | 143,8 |
| | a | | | 63,1 | | | | 117,9 | 122,1 | |
| | 29,7 | | | | | | | | | |

**TABLE II (follows)**

| Ex. Nº | Signal assignments:* 1 | 2 | 3 | 4 | 5 | 6 | 7 | 8 | 9 | 10 |
|---|---|---|---|---|---|---|---|---|---|---|
| 24 | 20,4<br>a<br>29,8 | 48,9 | 66,0 | 61,1<br>63,1 | 69,6 | 72,4 | 72,4 | 117,6<br>117,8 | 121,8<br>122,0 | 143,7<br>143,8 |
| 27 | 20,9<br>28,4 | 48,9 | 66,7 | 62,4<br>62,9 | 69,9 | 72,0 | 73,0 | 117,7<br>117,9 | 122,0<br>122,1 | 143,9<br>144,7 |
| 29 | 22,4<br>26,2<br>26,4<br>29,7 | 48,2 | 66,2 | 60,5<br>61,2 | 69,9 | 72,0 | 72,7 | 117,6<br>117,9 | 121,9<br>122,1 | 143,7 |
| 30 | 22,2<br>a<br>29,7 | 48,0 | 66,1 | 60,3<br>61,1 | 69,7 | 72,4 | 72,5 | 117,7<br>117,9 | 121,9<br>122,1 | 143,7 |
| 32 | 26,3<br>27,2<br>27,8<br>29,8 | 11,8 | 66,0 | 47,2<br>53,2 | 69,7 | 72,4 | 72,4 | 117,7<br>117,9 | 121,8<br>122,1 | 143,8<br>143,9 |

* 1: central $CH_2$ groups of the side chain; 2: $N-CH_3$; 3: benzodioxane $C^3H_2$; 4: $N-CH_2$; 5: benzodioxanyl-$CH_2$-O; 6: O-$CH_2$ chain; 7: benzodioxane $C_2H$; 8: benzodioxane $C^5H$ and $C^8H$; 9: benzodioxane $C^6H$ and $C^7H$; 10: benzodioxane $C^{4a}$ and $C^{8a}$.

**EXAMPLE 2**

**N-[5-[[(1,4-Benzodioxan-2-yl)methyl]oxy]pentyl]-N,N,N-trimethylammonium bromide (I, $R^1 = R^2 = R^3 = CH_3$, A = single bond, X = Br, m = 3, n = 2).**

a) 2-[[(5-Bromopentyl)oxy]methyl]-1,4-benzodioxane (II, A = single bond, X = Br, m = 3, n = 2).

Following the procedure described in Example 1a, but using 1,5-dibromopentane instead of 1,4-dibromobutane, the desired bromo-derivative is obtained in a 84% yield.

$^1H$ NMR (200 MHz, $CDCl_3$) δ(TMS): 1.48-1.97 (m, 6H, chain central $CH_2$), 3.44 (t, 2H, $CH_2Br$), 3.50-3.80 (m, 4H, $CH_2OCH_2$), 4.08 (dd, 1H, benzodioxane $C^2H$), 4.30 (m, 2H, benzodioxane $C^3H_2$), 6.89 (m, 4H,

benzodioxane $C^5H$-$C^8H$).

b) Preparation of the title compound.

Following the procedure described in Example 1b, starting from the bromo-derivative prepared in 2a, the title ammonium salt is obtained in a 90% yield, m.p. 159-160 °C. The most significant NMR data are reported in Tables I and II.

## EXAMPLE 3

**N-[6-[[(1,4-Benzodioxan-2-yl)methyl]oxy]hexyl]-N,N,N-trimethylammonium bromide (I, $R^1$ = $R^2$ = $R^3$ = $CH_3$, A = single bond, X = Br, m = 4, n = 2).**

a) 2-[[(6-Bromohexyl)oxy]methyl]-1,4-benzodioxane (II, A = single bond, X = Br, m = 4, n = 2).

Following the procedure described in Example 1a, but using 1,6-dibromohexane instead of 1,4-dibromobutane, the desired bromo-derivative is obtained in a 86% yield.

$^1H$ NMR (200 MHz, $CDCl_3$) δ(TMS): 1.35-1.91 (m, 8H, chain central $CH_2$), 3.34 (t, 2H, $CH_2Br$), 3.42-3.75 (m, 4H, $CH_2OCH_2$), 4.03 (dd, 1H, benzodioxane $C^2H$), 4.25 (m, 2H, benzodioxane $C^3H_2$), 6.83 (m, 4H, benzodioxane $C^5H$-$C^8H$).

b) Preparation of the title compound.

Following the procedure described in Example 1b, starting from the bromo-derivative prepared in 3a, the title ammonium salt is obtained in a 89% yield, m.p. 157-159 °C. The most significant NMR data are reported in Tables I and II.

## EXAMPLE 4

**N-[10-[[(1,4-Benzodioxan-2-yl)methyl]oxy]decyl]-N,N,N-trimethylammonium bromide (I, $R^1$ = $R^2$ = $R^3$ = $CH_3$, A = single bond, X = Br, m = 8, n = 2).**

a) 2-[[(10-Bromodecyl)oxy]methyl]-1,4-benzodioxane (II, A = single bond, X = Br, m = 8, n = 2).

Following the procedure described in Example 1a, but using 1,10-dibromodecane instead of 1,4-dibromobutane, the desired bromo-derivative is obtained in a 73% yield.

$^1H$ NMR (300 MHz, $CDCl_3$) δ(TMS): 1.28-1.85 (m, 16H, chain central $CH_2$), 3.37 (t, 2H, $CH_2Br$), 3.48-3.70 (m, 4H, $CH_2OCH_2$), 4.04 (dd, 1H, benzodioxane $C^2H$), 4.31 (m, 2H, benzodioxane $C^3H_2$), 6.85 (m, 4H, benzodioxane $C^5H$-$C^8H$).

b) Preparation of the title compound

Following the procedure described in Example 1b, starting from the bromo-derivative prepared in 4a, the title ammonium salt is obtained in a 83% yield, m.p. 164-165 °C. The most significant NMR data are reported in Tables I and II.

## EXAMPLE 5

**N-[16[[(1,4-Benzodioxan-2-yl)methyl]oxy]hexadecyl]-N,N,N-trimethylammonium bromide (I, $R^1$ = $R^2$ = $R^3$ = $CH_3$, A = single bond, X = Br, m = 8, n = 8).**

a) 2-[[(16-Bromohexadecyl)oxy]methyl]-1,4-benzodioxane (II, A = single bond, X = Br, m = 8, n = 8).

An ether solution of 6-[(1,4-benzodioxan-2-yl)methoxy]hexylmagnesium bromide is prepared starting from 3.29 g (0.01 mole) of 2-[[(6-bromohexyl)oxy]methyl]-1,4-benzodioxane and 0.243 g (0.01 mole) of magnesium in 80 ml of anhydrous tetrahydrofuran. The solution is cooled to 0 °C and slowly added to a thoroughly stirred solution of 1,10-dibromodecane (3 g, 0.01 mole) and $Li_2CuCl_4$ (0.001 mole) in 50 ml of anhydrous tetrahydrofuran, keeping temperature below 10 °C during the addition. The resulting suspension is left under stirring at room temperature for 18 hours, after which 200 ml of a saturated ammonium chloride solution is added. The reaction mixture is extracted with ethyl ether, the organic phases are washed with a saturated sodium chloride solution, dried over anhydrous sodium sulfate and evaporated to dryness. By chromatography on silica gel column, eluting with an hexane/ethyl ether 7:3 mixture, the title bromo-derivative is obtained, in a 55% yield.

$^1H$ NMR (300 MHz, $CDCl_3$) δ(TMS): 1.30 (broad signal, 24H, chain central $CH_2$), 1.57 (m, 2H, $CH_2$-C-Br), 1.83 (m, 2H, $CH_2$-C-O), 3.39 (t, 2H, $CH_2Br$), 3.48 (t, 2H, chain $CH_2O$), 3.64 (m, 2H, $CH_2O$), 4.04 (dd, 1H, benzodioxane $C^2H$), 4.32 (m, 2H, benzodioxane $C^3H_2$), 6.84 (m, 4H, benzodioxane $C^5H$-$C^8H$).

b) Preparation of the title compound.

Following the procedure described in Example 1b, starting from the bromo-derivative prepared in 5a, the title ammonium salt is obtained in a 91% yield, m.p. 184-186 ° C. The most significant NMR data are reported in Table I.

**EXAMPLE 6**

**N-[6-[[(10-[[1,4-Benzodioxan-2-yl)methyl]oxy]decyl]oxy]hexyl]-N,N,N-trimethylammonium bromide (I, $R^1 = R^2 = R^3 = CH_3$, A = O, X = Br, m = 10, n = 6).**

a) 10-[[(1,4-Benzodioxan-2-yl)methyl]oxy]decane-1-ol (VI, A-P = OH, m = 10).

3.85 g (0.01 mole) of 2-[[(10-bromodecyl)oxy]methyl]-1,4-benzodioxane(obtained according to Example 4a) and 3.5 g (0.05 mole) of anhydrous sodium acetate are dissolved in 100 ml of anhydrous dimethylformamide. The reaction mixture is stirred and heated to 80-90 ° C for 18 hours, after that it is poured onto an ice-water excess and extracted with ethyl ether (3 x 60 ml). The organic phases are washed with a sodium chloride saturated solution, dried over anhydrous sodium sulfate and evaporated to dryness. The residue is dissolved in 100 ml of methanol, then added with 7 g (0.1 mole) of 80% potassium hydroxide in 30 ml of water. The resulting mixture is refluxed for two hours, then cooled and evaporated to 1/4th of the original volume, finally it is diluted with 100 ml of water. The solution is extracted with ethyl ether, the organic phases are washed with a sodium chloride saturated solution, evaporated to dryness and the residue is subjected to chromatography on silica gel column, eluting with an hexane/ethyl ether 3:1 mixture, to obtain the title alcohol in a 91% yield.

b) 2-[[[10-[(6-Bromohexyl)oxy]decyl]oxy]methyl]-1,4-benzodioxane (II, A = O, X = Br, m = 10, n = 6).

Starting from the alcohol prepared in 6a and 1,6-dibromohexane, following the procedure described in Example 4a, bromo-derivative 6b is obtained in a 63% yield.

$^1H$ NMR (300 MHz, $CDCl_3$) $\delta$(TMS): 1.20-1.32 (broad signal, 22H, chain central $CH_2$), 1.50 (m, 4H, $CH_2$-C-O), 1.86 (m, 2H, $CH_2$-C-Br), 3.34-3.68 (complex system, 6H, $CH_2$-O and $CH_2Br$), 4.00 (dd, 1H, benzodioxane $C^2H$), 4.22 (m, 2H, benzodioxane $C^3H_2$), 6.8 (m, aromatic 4H).

c) Preparation of the title compound.

Following the procedure described in Example 1b, starting from the bromo-derivative prepared in 6b, the title ammonium salt is obtained in a 70% yield, m.p. 99-100 ° C.

**EXAMPLE 7**

**N-[10-[[10-[[(1,4-Benzodioxan-2-yl)methyl]oxy]decyl]oxy]decyl]-N,N,N-trimethylammonium bromide (I, $R^1 = R^2 = R^3 = CH_3$, A = O, X = Br, m = n = 10).**

a) 2-[[[10-[(10-Bromodecyl)oxy]decyl]oxy]methyl]-1,4-benzodioxane (II, A = O, X = Br, m = n = 10).

Starting from the alcohol prepared in Example 6a and 1,10-dibromodecane, following the procedure described in Example 4a, the desired bromo-derivative is obtained in a 79% yield.

$^1H$ NMR (300 MHz, $CDCl_3$) $\delta$(TMS): 1.14-1.32 (broad signal, chain central $CH_2$), 1.33-1.58 (broad signal, 4H, $CH_2$-C-O), 3.34-3.68 (complex system, 6H, $CH_2$-O and $CH_2$-Br), 4.00 (dd, 1H, benzodioxane $C^2H$), 4.22 (m, 2H, benzodioxane $C^3H_2$), 6.8 (m, aromatic 4H).

b) Preparation of the title compound.

Following the procedure described in Example 1b, starting from the bromo-derivative prepared in 7a, the title ammonium salt is obtained in a 76% yield, m.p. 165-166 ° C.

**EXAMPLE 8**

**N-[4-[[(1,4-Benzodioxan-2-yl)methyl]oxy]butyl]-N,N,N-triethylammonium bromide (I, $R^1 = R^2 = R^3 = CH_2CH_3$, A = single bond, X = Br, m = n = 2).**

6.02 g (0.02 mole) of 2-[[(4-bromobutyl)oxy]methyl]-1,4-benzodioxane,obtained according to the procedure described in Example 1a, are dissolved in 50 ml of absolute ethanol and 20 ml of distilled triethylamine. The solution is refluxed for 12 hours, thereafter a second portion of 10 ml of triethylamine is added and heating is continued for 12 more hours. The resulting solution is cooled, evaporated to dryness and the residue is crystallized from an anhydrous acetone/triethylamine mixture to obtain the title ammonium salt in a 88% yield, in form of a white solid, m.p. 60-62 ° C. The most significant NMR data are reported in Tables I and II.

**EXAMPLE 9**

**N-[6-[[(1,4-Benzodioxan-2-yl)methyl]oxy]hexyl]-N,N,N-triethylammonium bromide (I, $R^1$ = $R^2$ = $R^3$ = $CH_2CH_3$, A = single bond, X = Br, m = 4, n = 2).**

Following the procedure described in Example 8, starting from the bromo-derivative prepared in Example 3a, the title ammonium salt is obtained in a 87% yield, m.p. 64-66 °C. The most significant NMR data are reported in Tables I and II.

**EXAMPLE 10**

**N-[10-[[(1,4-Benzodioxan-2-yl)methyl]oxy]decyl]-N,N,N-triethylammonium bromide (I, $R^1$ = $R^2$ = $R^3$ = $CH_2CH_3$, A = single bond, X = Br, m = 8, n = 2).**

Following the procedure described in Example 8, starting from the bromo-derivative prepared in Example 4a, the title ammonium salt is obtained in a 85% yield, m.p. 52-53 °C. The most significant NMR data are reported in Tables I and II.

**EXAMPLE 11**

**N-[16-[[(1,4-Benzodioxan-2-yl)methyl]oxy]hexadecyl]-N,N,N-triethylammonium bromide (I, $R^1$ = $R^2$ = $R^3$ = $CH_2CH_3$, A = single bond, X = Br, m = n = 8).**

Following the procedure described in Example 8, starting from the bromo-derivative prepared in Example 5a, the title ammonium salt is obtained in a 82% yield, m.p. 67-69 °C. The most significant NMR data are reported in Table I.

**EXAMPLE 12**

**N-[4-[[(1,4-Benzodioxan-2-yl)methyl]oxy]butyl]piperidine hydrochloride (I, $R^1$ = H, $R^2$, $R^3$ = $(CH_2)_5$, A = single bond, X = Cl, m = n = 2).**

3.01 g (0.01 mole) of 2-[[(4-bromobutyl)oxy]methyl]-1,4-benzodioxane,obtained as described in Example 1a, are dissolved in 15 ml of distilled piperidine. The solution is refluxed for 4 hours, then it is evaporated to dryness and the residue is subjected to chromatography on silica gel column, eluting with an ethyl acetate/ethanol/diethylamine 95:3:2 mixture, to obtain the title amine as the free base in a 84% yield. From this amine the hydrochloride is prepared which, by crystallization from an anhydrous acetone/ethanol mixture, gives a white solid, m.p. 110-113 °C. The most significant NMR data of the title amine are reported in Tables I and II.

**EXAMPLE 13**

**N-[5-[[(1,4-Benzodioxan-2-yl)methyl]oxy]pentyl]piperidine hydrochloride (I, $R^1$ = H, $R^2$, $R^3$ = $(CH_2)_5$, A = single bond, X = Cl, m = 3, n = 2).**

Following the procedure described in Example 12, starting from the bromo-derivative prepared in Example 2a, the title amine is obtained as the free base in a 90% yield. From this amine the hydrochloride is prepared which, by crystallization from anhydrous acetone, gives a white solid, m.p. 134-135 °C. The most significant NMR data are reported in Table I.

**EXAMPLE 14**

**N-[6-[[(1,4-Benzodioxan-2-yl)methyl]oxy]hexyl]piperidine hydrochloride (I, $R^1$ = H, $R^2$, $R^3$ = $(CH_2)_5$, A = single bond, X = Cl, m = 4, n = 2).**

Following the procedure described in Example 12, starting from the bromo-derivative prepared in Example 3a, the title amine is obtained as the free base in a 88% yield. From this amine the hydrochloride is prepared which, by crystallization from anhydrous acetone, gives a white solid, m.p. 108-110 °C. The

most significant NMR data of the title amine are reported in Tables I and II.

**EXAMPLE 15**

**N-[10-[[(1,4-Benzodioxan-2-yl)methyl]oxy]decyl]piperidine hydrochloride (I, $R^1$ = H, $R^2$, $R^3$ = $(CH_2)_5$, A = single bond, X = Cl, m = 8, n = 2).**

Following the procedure described in Example 12, starting from the bromo-derivative prepared in Example 4a, the title amine is obtained as the free base, in a 87% yield. From this amine the hydrochloride is prepared which, by crystallization from an anhydrous acetone/ethyl ether mixture, gives a white solid, m.p. 121-122 °C. The most significant NMR data of the title amine are reported in Tables I and II.

**EXAMPLE 16**

**N-[4-[[(1,4-Benzodioxan-2-yl)methyl]oxy]butyl]morpholine hydrochloride (I, $R^1$ = H, $R^2$, $R^3$ = $(CH_2)_2O(CH_2)_2$, A = single bond, X = Cl, m = n = 2).**

Following the procedure described in Example 12, starting from the bromo-derivative prepared in Example 1a and using morpholine instead of piperidine, the title amine is obtained as the free base, in a 92% yield. From this amine the hydrochloride is prepared which, by crystallization from an anhydrous acetone/ethanol mixture, gives a white solid, m.p. 151-152 °C. The most significant NMR data of the title amine are reported in Tables I and II.

**EXAMPLE 17**

**N-[5-[[(1,4-Benzodioxan-2-yl)methyl]oxy]pentyl]morpholine hydrochloride (I, $R^1$ = H, $R^2$, $R^3$ = $(CH_2)_2O(CH_2)_2$, A = single bond, X = Cl, m = 3, n = 2).**

Following the procedure described in Example 12, starting from the bromo-derivative prepared in Example 2a and using morpholine instead of piperidine, the title amine is obtained as the free base in a 88% yield. From this amine the hydrochloride is prepared which, by crystallization from an anhydrous acetone/ethanol mixture, gives a white solid, m.p. 139-140 °C. The most significant NMR data are reported in Table I.

**EXAMPLE 18**

**N-[6-[[(1,4-Benzodioxan-2-yl)methyl]oxy]hexyl]morpholine hydrochloride (I, $R^1$ = H, $R^2$, $R^3$ = $(CH_2)_2O(CH_2)_2$, A = single bond, X = Cl, m = 4, n = 2).**

Following the procedure described in Example 12, starting from the bromo-derivative prepared in Example 3a and using morpholine instead of piperidine, the title amine is obtained as the free base in a 95% yield. From this amine the hydrochloride is prepared which, by crystallization from an anhydrous acetone/ethanol mixture, gives a white solid, m.p. 103-105 °C. The most significant NMR data of the title amine are reported in Tables I and II.

**EXAMPLE 19**

**N-[10-[[(1,4-Benzodioxan-2-yl)methyl]oxy]decyl]morpholine hydrochloride (I, $R^1$ = H, $R^2$, $R^3$ = $(CH_2)_2O(CH_2)_2$, A = single bond, X = Cl, m = 8, n = 2).**

Following the procedure described in Example 12, starting from the bromo-derivative prepared in Example 4a and using morpholine instead of piperidine, the title amine is obtained as the free base in a 95% yield. From this amine the hydrochloride is prepared which, by crystallization from anhydrous acetone, gives a white solid, m.p. 119-122 °C. The most significant NMR data of the title amine are reported in Tables I and II.

**EXAMPLE 20**

**N-[4-[[(1,4-Benzodioxan-2-yl)methyl]oxy]butyl]-N-methylpiperidinium iodide (I, $R^1$ = $CH_3$, $R^2$, $R^3$ = $(CH_2)_5$, A = single bond, X = I, m = 2, n = 2).**

A solution of 3.05 g (0.01 mole) of N-[4-[[(1,4-benzodioxan-2-yl)methyl]oxy]butyl]piperidine (obtained as described in Example 12) as the free base, in 10 ml anhydrous acetone and 30 ml anhydrous toluene is prepared. A solution of 14 g (0.1 mole) of methyl iodide in 10 ml of anhydrous toluene is dropped into the above solution. The reaction mixture is stirred at room temperature for 24 hours, then the resulting solid is recovered by filtration and crystallized from an anhydrous acetone/absolute ethanol mixture, to obtain a slightly yellow solid, m.p. 129-130 °C. The most significant NMR data of the title amine are reported in Tables I and II.

**EXAMPLE 21**

**N-[5-[[(1,4-Benzodioxan-2-yl)methyl]oxy]pentyl]-N-methylpiperidinium iodide (I, $R^1$ = $CH_3$, $R^2$, $R^3$ = $(CH_2)_5$, A = single bond, X = I, m = 3, n = 2).**

Following the procedure described in Example 20, starting from the amine prepared in Example 13, the title compound is obtained in a 82% yield. This compound, by crystallization from an anhydrous acetone/absolute ethanol mixture, gives a slightly yellow solid, m.p. 120-121 °C. The most significant NMR data of the title amine are reported in Tables I and II.

**EXAMPLE 22**

**N-[6-[[(1,4-Benzodioxan-2-yl)methyl]oxy]hexyl]-N-methylpiperidinium iodide (I, $R^1$ = $CH_3$, $R^2$, $R^3$ = $(CH_2)_5$, A = single bond, X = I, m = 4, n = 2).**

Following the procedure described in Example 20, starting from the amine prepared in Example 14, the title compound is obtained in a 85% yield. This compound, by crystallization from an anhydrous acetone/absolute ethanol mixture, gives a white solid, m.p. 120-121.5 °C. The most significant NMR data of the title amine are reported in Tables I and II.

**EXAMPLE 23**

**N-[10-[[(1,4-Benzodioxan-2-yl)methyl]oxy]decyl]-N-methylpiperidinium bromide (I, $R^1$ = $CH_3$, $R^2$, $R^3$ = $(CH_2)_5$, A = single bond, X = Br, m = 8, n = 2).**

Following the procedure described in Example 12, starting from the bromo-derivative prepared in Example 4a and using N-methylpiperidine instead of piperidine, the title compound is obtained in a 78% yield. This compound, by crystallization from anhydrous acetone, gives a white solid, m.p. 104-105 °C. The most significant NMR data are reported in Tables I and II.

**EXAMPLE 24**

**N-[16-[[(1,4-Benzodioxan-2-yl)methyl]oxy]hexadecyl]-N-methylpiperidinium bromide (I, $R^1$ = $CH_3$, $R^2$, $R^3$ = $(CH_2)_5$, A = single bond, X = Br, m = 8, n = 8).**

Following the procedure described in Example 12, starting from the bromo-derivative prepared in Example 5a and using N-methylpiperidine instead of piperidine, the title compound is obtained in a 72% yield. This compound, by crystallization from anhydrous acetone, gives a white solid, m.p. 140-141 °C. The most significant NMR data of the title amine are reported in Tables I and II.

**EXAMPLE 25**

**N-[6-[[(10-[[1,4-Benzodioxan-2-yl)methyl]oxy]decyl]oxy]hexyl]-N-methylpiperidinium bromide (I, $R^1$ = $CH_3$, $R^2$, $R^3$ = $(CH_2)_5$, A = O, X = Br, m = 10, n = 6).**

Following the procedure described in Example 12, starting from the bromo-derivative prepared in Example 6b, using N-methylpiperidine instead of piperidine, the title compound is obtained in a 80% yield. This compound, by crystallization from anhydrous acetone, gives a white-yellowish solid, m.p. 73-74 °C.

**EXAMPLE 26**

**N-[10-[[10-[[(1,4-Benzodioxan-2-yl)methyl]oxy]decyl]oxy]decyl]-N-methylpiperidinium bromide (I, $R^1$ = $CH_3$, $R^2$, $R^3$ = $(CH_2)_5$, A = O, X = Br, m = n = 10).**

Following the procedure described in Example 12, starting from the bromo-derivative prepared in Example 7a and using N-methylpiperidine instead of piperidine, the title compound is obtained in a 80% yield. This compound, by crystallization from anhydrous acetone, gives a white-yellowish solid, m.p. 106-107 °C.

**EXAMPLE 27**

**N-[4-[[(1,4-Benzodioxan-2-yl)methyl]oxy]butyl]-N-methylmorpholinium iodide (I, $R^1$ = $CH_3$, $R^2$, $R^3$ = $(CH_2)_2O(CH_2)_2$, A = single bond, X = I, m = 2, n = 2).**

Following the procedure described in Example 20, starting from the amine prepared in Example 16, the title compound is obtained in a 89% yield. This compound, by crystallization from an anhydrous acetone/absolute ethanol mixture, gives a white solid, m.p. 160-161 °C. The most significant NMR data of the title amine are reported in Tables I and II.

**EXAMPLE 28**

**N-[5-[[(1,4-Benzodioxan-2-yl)methyl]oxy]pentyl]-N-methylmorpholinium iodide (I, $R^1$ = $CH_3$, $R^2$, $R^3$ = $(CH_2)_2O(CH_2)_2$, A = single bond, X = I, m = 3, n = 2).**

Following the procedure described in Example 20, starting from the amine prepared in Example 17, the title compound is obtained in a 83% yield. This compound, by crystallization from an anhydrous acetone/absolute ethanol mixture, gives a white solid, m.p. 133-135 °C. The most significant NMR data are reported in Table I.

**EXAMPLE 29**

**N-[6-[[(1,4-Benzodioxan-2-yl)methyl]oxy]hexyl]-N-methylmorpholinium iodide (I, $R^1$ = $CH_3$, $R^2$, $R^3$ = $(CH_2)_2O(CH_2)_2$, A = single bond, X = I, m = 4, n = 2).**

Following the procedure described in Example 20, starting from the amine prepared in Example 18, the title compound is obtained in a 88% yield. This compound, by crystallization from an anhydrous acetone/absolute ethanol mixture, gives a white solid, m.p. 142-143 °C. The most significant NMR data of the title amine are reported in Tables I and II.

**EXAMPLE 30**

**N-[10-[[(1,4-Benzodioxan-2-yl)methyl]oxy]decyl]-N-methylmorpholinium iodide (I, $R^1$ = $CH_3$, $R^2$, $R^3$ = $(CH_2)_2O(CH_2)_2$, A = single bond, X = I, m = 8, n = 2).**

Following the procedure described in Example 20, starting from the amine prepared in Example 19, the title compound is obtained in a 84% yield. This compound, by crystallization from an anhydrous acetone/ethyl ether mixture, gives a white solid, m.p. 112-114 °C. The most significant NMR data of the title amine are reported in Tables I and II.

## EXAMPLE 31

**N-[4-[[(1,4-Benzodioxan-2-yl)methyl]oxy]butyl]-N,N-diethylamine hydrochloride (I, $R^1$ = H, $R^2$, $R^3$ = $CH_2CH_3$, A = single bond, X = Cl, m = n = 2).**

3.01 g (0.01 mole) of 2-[[(4-bromobutyl)oxy]methyl]-1,4-benzodioxane,obtained as described in Example 1a, are dissolved in 15 ml of distilled diethylamine. The solution is refluxed for 6 hours, after that it is evaporated to dryness and the residue is subjected to chromatography on silica gel column, eluting with an ethyl acetate/diethylamine 98:2 mixture, to obtain the title amine as the free base, in a 88% yield. From this amine the hydrochloride is prepared which, by crystallization from an anhydrous acetone/ethanol mixture, gives a white solid, m.p. 93-94°C. The most significant NMR data are reported in Table I.

## EXAMPLE 32

**N-[6-[[(1,4-Benzodioxan-2-yl)methyl]oxy]hexyl]-N,N-diethylamine hydrochloride (I, $R^1$ = H, $R^2$, $R^3$ = $CH_2CH_3$, A = single bond, X = Cl, m = 4, n = 2).**

Following the procedure described in Example 31, starting from the bromo-derivative prepared in Example 3a, the title amine is obtained as the free base in a 86% yield. From this amine the hydrochloride is prepared which, by crystallization from anhydrous acetone, gives a white solid, m.p. 90-92°C. The most significant NMR data of the title amine are reported in Tables I and II.

## EXAMPLE 33

**N-[10-[[(1,4-Benzodioxan-2-yl)methyl]oxy]decyl]N,N-diethylamine hydrochloride (I, $R^1$ = H, $R^2$, $R^3$ = $CH_2CH_3$, A = single bond, X = Cl, m = 8, n = 2).**

Following the procedure described in Example 31, starting from the bromo-derivative prepared in Example 4a, the title amine is obtained as the free base in a 85% yield. From this amine the hydrochloride is prepared which, by crystallization from anhydrous acetone, gives a white solid. The most significant NMR data of the title amine are reported in Table I.

## EXAMPLE 34

**N-[16-[[(1,4-Benzodioxan-2-yl)methyl]oxy]hexadecyl]piperidine hydrochloride (I, $R^1$ = H, $R^2$, $R^3$ = $(CH_2)_5$, A = single bond, X = Cl, m = n = 8).**

Following the procedure described in Example 12, starting from the bromo-derivative prepared in Example 5a, the title amine is obtained as the free base in a 83% yield. From this amine the hydrochloride is prepared which, by crystallization from an anhydrous acetone/ethyl ether mixture, gives a white solid, m.p. 129-130°C. The most significant NMR data of the title amine are reported in Table I.

## EXAMPLE 35

**N-[10-[[10-[[(1,4-Benzodioxan-2-yl)methyl]oxy]decyl]oxy]decyl]piperidine hydrochloride (I, $R^1$ = H, $R^2$, $R^3$ = $(CH_2)_5$, A = O, X = Cl, m = n = 10).**

Following the procedure described in Example 12, starting from the bromo-derivative prepared in Example 7a, the title amine is obtained as the free base in a 87% yield. From this amine the hydrochloride is prepared which, by crystallization from an anhydrous acetone/ethyl ether mixture, gives a white solid, m.p. 113-114°C.

## EXAMPLE 36

**(2S)-N-[10-[[(1,4-Benzodioxan-2-yl)methyl]oxy]decyl]piperidine hydrochloride (I, $R^1$ = H, $R^2$, $R^3$ = $(CH_2)_5$, A = single bond, X = Cl, m = 8, n = 2).**

a) (2S)-2-[[(10-Bromodecyl)oxy]methyl]-1,4-benzodioxane (II, A = single bond, X = Br, m = 8, n = 2).

Following the procedure described in Example 1a, but using 1,10-dibromodecane instead of 1,4-dibromobutane, and (2S)-2-hydroxymethyl-1,4-benzodioxane, $[\alpha]_D$ = -34° (c=0.6; ethanol), the title bromo-derivative is obtained in a 90% yield. This compound has specific rotatory power $[\alpha]_D$ = -13.4° (c=0.6; ethanol) and the same $^1H$ NMR spectrum (300 MHz, $CDCl_3$) as that of the bromo-derivative obtained in Example 4a.

b) Following the procedure described in Example 12, starting from the bromo-derivative prepared in 36a, the title amine is obtained as the free base, having specific rotatory power $[\alpha]_D$ = +11.7° (c=0.45; ethanol), in a 83% yield. From this amine the hydrochloride is prepared which, by crystallization from an anhydrous ethyl acetate/ethyl ether mixture, gives a white solid, m.p. 118.5-119.0°C. The NMR spectra of the title amine are the same as those of the compound prepared in Example 15.

## EXAMPLE 37

**(2R)-N-[10-[[(1,4-Benzodioxan-2-yl)methyl]oxy]decyl]piperidine hydrochloride (I, $R^1$ = H, $R^2$, $R^3$ = $(CH_2)_5$, A = single bond, X = Cl, m = 8, n = 2).**

a) (2R)-2-[[(10-Bromodecyl)oxy]methyl]-1,4-benzodioxane (II, A = single bond, X = Br, m = 8, n = 2).

Following the procedure described in Example 1a, but using 1,10-dibromodecane instead of 1,4-dibromobutane, and (2R)-2-hydroxymethyl-1,4-benzodioxane, having $[\alpha]_D$ = +34° (c=0.6; ethanol), the title bromo-derivative is obtained in a 90% yield. This compound has specific rotatory power $[\alpha]_D$ = +13.4° (c=0.6; ethanol) and the same $^1H$ NMR spectrum (300 MHz, $CDCl_3$) as that of the bromo-derivative obtained in Example 4a.

b) Following the procedure described in Example 12, starting from the bromo-derivative prepared in 37a, the title amine is obtained as the free base, having specific rotatory power $[\alpha]_D$ = -11.7° (c=0.45; ethanol), in a 83% yield. From this amine the hydrochloride is prepared which, by crystallization from an anhydrous ethyl acetate/ethyl ether mixture, gives a white solid, m.p. 119-119.5°C. The NMR spectra of the title amine are the same as those of the compound prepared in Example 15.

## EXAMPLE 38

Evaluation of the anti-platelet action

Platelets are prepared starting from blood samples obtained by means of a carotid cannula from male albino New Zealand rabbits (2-2.5 kg), subjected to anticoagulant treatment with sodium citrate dihydrate (3.2%) 1:10. Blood is centrifuged (150xg, 10 min, 22°C) to separate the platelet-rich plasma fraction (PRP) from erythrocytes. Platelets are separated from plasmatic components by serial washings and centrifugations (Blood, 1986, 62:337), thereafter they are suspended in a physiological buffer at a concentration of 5 x $10^5$/ml. A 400 µl volume of the above mentioned suspension is placed into the cuvette of a multicanal aggregometer (Aggrecoder HU) where it is kept at constant temperature and stirring. After stabilization, platelets are added with 50 µl of physiological buffer containing 100 µM acetylsalicylic acid and apirase 5 mg/ml, in order to block any non PAF-related platelet activation metabolic pathways. Subsequently the platelet suspensions are added with 50 µl of the different solutions to be tested. Changes in light transmission, due to platelet aggregation in the suspension, are recorded and expressed as percent platelet aggregation, according to the procedure by Born and Cross (J. Physiol., 1962, 162:67). Standard curve is performed with PAF within a concentration range of 0.1-1 nM.

The results from the tests in which PAF activity was measured are reported in Table III.

TABLE III

| Platelet aggregating effect and inhibition PAF-induced platelet aggregation. | | |
|---|---|---|
| Compound of Example n° | Aggregation $EC_{50}$ (μM) | Paf-antagonism $IC_{50}$ (μM) |
| 4 | - | 130 |
| 5 | - | 20 |
| 8 | >190 | - |
| 10 | - | 90 |
| 21 | >190 | - |
| 23 | - | 75 |
| 30 | - | 190 |

**EXAMPLE 39**

Inhibition of PAF-induced platelet aggregation

Capability to inhibit PAF-induced platelet aggregation is evaluated by means of the aggregometry technique in suspensions of rabbit washed platelets, obtained by means of the same procedure as described in Example 32. For this purpose, the inhibition potency of the products under test, expressed as the corresponding $IC_{50}$, is evaluated on the maximum aggregation values obtained in PAF standard curve. The results obtained from the tests, in which PAF-antagonist activity was measured, are reported in Table III.

**EXAMPLE 40**

Evaluation of cytotoxic activity

Suspensions of HL-60 Line cells, obtained starting from human promyelocyte leukemia, are incubated in 6-well culture plates for 24 hours at 37°C, at a concentration of 1 x $10^6$ cells/ml, in the presence of the test products, which have previously been dissolved in the culture medium. Cytotoxicity is measured by means of the Trypan Blue exclusion technique (Practical Immunology, pp 29-32, Oxford, England : Blackwell Scientific Publications, 1976). Cytotoxic activities of the products, expressed as $IC_{50}$, are reported in Table IV.

TABLE IV

| Cytotoxic effect on HL-60 tumor cells. | |
|---|---|
| Compound of Example n° | Cytotoxicity $EC_{50}$ (μM) |
| 4 | 70 |
| 5 | 18 |
| 6 | 11 |
| 7 | 15 |
| 10 | 30 |
| 15 | 20 |
| 16 | 18 |
| 19 | 30 |
| 23 | 20 |
| 24 | 6 |
| 25 | 3 |
| 26 | 28 |
| 30 | 30 |

## Claims

**Claims for the following Contracting States : AT, BE, CH, DE, DK, FR, GB, IT, LI, LU, NL, SE**

1. Compounds of formula (I)

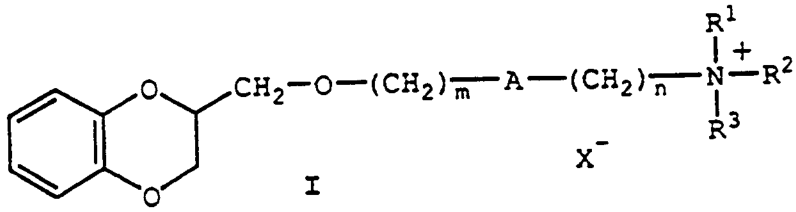

I

wherein $R^1$, $R^2$ and $R^3$, which can be the same or different, are hydrogen, straight or branched $C_1$-$C_6$ alkyl, aryl or aralkyl, in which the alkyl portion is straight or branched $C_1$-$C_6$ alkyl;
or $R^1R^2R^3N^+$ is an aromatic cyclic ammonium group, selected from 1-pyridinium, 1-quinolinium, 2-isoquinolinium, 1-imidazolium, 1-pyrazolium, 3-thiazolium, 3-oxazolium, 1-benzimidazolium, 3-benzothiazolium or 3-benzoxazolium, having on its ring from 1 to 3 substituents, selected from straight or branched $C_1$-$C_6$ alkyl, halogen, hydroxyalkyl in which the alkyl portion is straight or branched $C_1$-$C_6$ alkyl, amino, aminoalkyl in which the alkyl portion is straight or branched $C_1$-$C_6$ alkyl, or carboxy;
or $R^1R^2R^3N^+$ is a non aromatic cyclic ammonium group, in which two of groups ($R^1$, $R^2$ or $R^3$), together with the nitrogen atom, form a ring selected from 1-pyrrolidine, 1-piperidine, 4-morpholine, 1-piperazine or 1-perhydroazepine and the remaining group is hydrogen or straight or branched $C_1$-$C_6$ alkyl;
m and n, which can be the same or different, are an integer from 2 to 12;
A is either a single covalent bond or it is an oxygen atom;
$X^-$ is a pharmaceutically acceptable anion,
stereoisomers of compounds (I) and the mixtures thereof, and pharmaceutically acceptable salts thereof.

2. Compounds as claimed in claim 1, wherein m + n = 7-22.

3. Compounds according to claim 1 or 2, wherein $R^1$, $R^2$ and $R^3$ are hydrogen, straight or branched $C_1$-$C_6$ alkyl, aryl or aralkyl, in which the alkyl portion is straight or branched $C_1$-$C_6$ alkyl.

4. Compounds according to claim 1 or 2, wherein $R^1R^2R^3N^+$ is an aromatic cyclic ammonium group selected from 1-pyridinium, 1-imidazolium or 3-thiazolium.

5. Compounds according to claim 1 or 2, wherein $R^1R^2R^3N^+$ is a non aromatic cyclic ammonium group selected from 1-pyrrolidinium, 1-piperidinium, 4-morpholinium, 1-methyl-1-pyrrolinium, 1-methyl-1-piperidinium or 4-methyl-4-morpholinium.

6. Compounds according to any one of claims 1-5, selected from:
N-[10-[[(1,4-benzodioxan-2-yl)methyl]oxy]decyl]-N,N,N-trimethylammonium,
N-[16-[[(1,4-benzodioxan-2-yl)methyl]oxy]hexadecyl]-N,N,N-trimethylammonium,
N-[6-[[10-[[(1,4-benzodioxan-2-yl)methyl]oxy]decyl]oxy]hexyl]-N,N,N-trimethylammonium,
N-[10-[[10-[[(1,4-benzodioxan-2-yl)methyl]oxy]decyl]oxy]decyl]-N,N,N-trimethylammonium,
N-[10-[[(1,4-benzodioxan-2-yl)methyl]oxy]decyl]-N,N,N-triethylammonium,
N-[16-[[(1,4-benzodioxan-2-yl)methyl]oxy]hexadecyl]-N,N,N-triethylammonium,
N-[10-[[(1,4-benzodioxan-2-yl)methyl]oxy]decyl]-N-methylpiperidinium,
N-[16-[[(1,4-benzodioxan-2-yl)methyl]oxy]hexadecyl]-N-methylpiperidinium,
N-[6-[[10-[[(1,4-benzodioxan-2-yl)methyl]oxy]decyl]oxy]hexyl]-N-methylpiperidinium,
N-[10-[[10-[[(1,4-benzodioxan-2-yl)methyl]oxy]decyl]oxy]decyl]-N-methylpiperidinium,
N-[10-[[(1,4-benzodioxan-2-yl)methyl]oxy]decyl]-N-methylmorpholinium,
N-[10-[[(1,4-benzodioxan-2-yl)methyl]oxy]decyl]piperidinium,
N-[16-[[(1,4-benzodioxan-2-yl)methyl]oxy]hexadecyl]piperidinium,
N-[10-[[6-[[(1,4-benzodioxan-2-yl)methyl]oxy]decyl]oxy]hexyl]piperidinium,
N-[10-[[10-[[(1,4-benzodioxan-2-yl)methyl]oxy]decyl]oxy]decyl]piperidinium,

N-[10-[[(1,4-benzodioxan-2-yl)methyl]oxy]decyl]morpholinium,
(2S)-N-[10-[[(1,4-benzodioxan-2-yl)methyl]oxy]decyl]piperidinium,
(2R)-N-[10-[[(1,4-benzodioxan-2-yl)methyl]oxy]decyl]piperidinium, wherein $X^-$ is as defined in claim 1

**7.** A process for the preparation of the compounds claimed in claims 1 to 6, in which process a compound of formula (II)

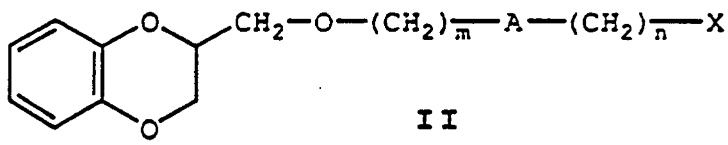

II

wherein X is an appropriate leaving group, and A, m and n have the above mentioned meanings, is reacted with a nitrogen base of formula $NR^1R^2R^3$ wherein $R^1$, $R^2$ and $R^3$ have the above mentioned meanings.

**8.** A process according to claim 7, in which process the reaction is carried out in the nitrogen base $NR^1R^2R^3$ itself as the solvent.

**9.** Pharmaceutical compositions containing as the active principle a compound of claims 1-6 in admixture with a suitable carrier.

**Claims for the following Contracting States : ES, GR**

**1.** A process for the preparation of compounds of formula (I) and pharmaceutically acceptable salts thereof

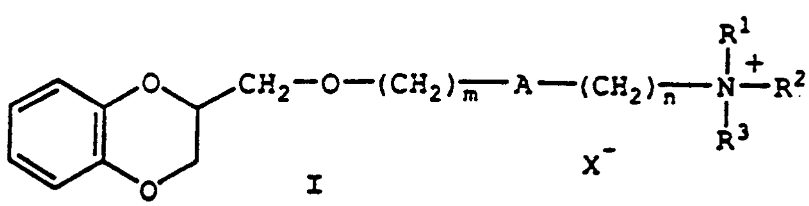

I

wherein $R^1$, $R^2$ and $R^3$, which can be the same or different, are hydrogen, straight or branched $C_1$-$C_6$ alkyl, aryl or aralkyl, in which the alkyl portion is straight or branched $C_1$-$C_6$ alkyl;
or $R^1R^2R^3N^+$ is an aromatic cyclic ammonium group, selected from 1-pyridinium, 1-quinolinium, 2-isoquinolinium, 1-imidazolium, 1-pyrazolium, 3-thiazolium, 3-oxazolium, 1-benzimidazolium, 3-benzothiazolium or 3-benzoxazolium, having on its ring from 1 to 3 substituents, selected from straight or branched $C_1$-$C_6$ alkyl, halogen, hydroxyalkyl in which the alkyl portion is straight or branched $C_1$-$C_6$ alkyl, amino, aminoalkyl in which the alkyl portion is straight or branched $C_1$-$C_6$ alkyl, or carboxy;
or $R^1R^2R^3N^+$ is a non aromatic cyclic ammonium group, in which two of groups ($R^1$, $R^2$ or $R^3$), together with the nitrogen atom, form a ring selected from 1-pyrrolidine, 1-piperidine, 4-morpholine, 1-piperazine or 1-perhydroazepine and the remaining group is hydrogen or straight or branched $C_1$-$C_6$ alkyl;
m and n, which can be the same or different, are an integer from 2 to 12;
A is either a single covalent bond or it is an oxygen atom;
$X^-$ is a pharmaceutically acceptable anion,
characterized in that a compound of formula (II)

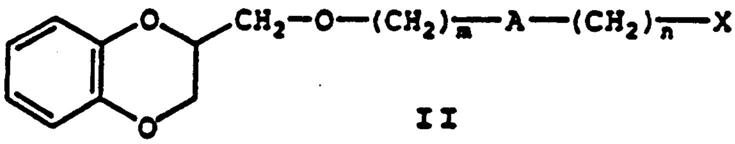

II

wherein X is an appropriate leaving group, is reacted with the nitrogen base $R^1R^2R^3N$, wherein $R^1$, $R^2$ and $R^3$ have the above defined meaning.

2. A process according to claim 1, characterized in that, when one of the groups $R^1$, $R^2$ and $R^3$ is hydrogen, said compound I will be subjected optionally to a second reaction with a compound of formula $R^1X$, in which $R^1$ is a straight or branched $C_1$-$C_6$ alkyl alkyl group or an aralkyl group in which the alkyl portion is straight or branched $C_{1-6}$ alkyl, and X represents the above mentioned groups, to obtain a compound I in which $R^1$ is different from hydrogen.

3. A process according to claim 1, characterized in that the sum of the values m and n is from 7 to 22.

4. A process according to claim 3, characterized in that $R^1$, $R^2$ and $R^3$ are hydrogen, $C_1$-$C_6$ alkyl, aryl or aralkyl groups, in which the alkyl portion is $C_1$-$C_6$ alkyl.

5. A process according to claim 4, characterized in that $R^1$, $R^2$ and $R^3$ are methyl or ethyl groups.

6. A process according to claim 3, characterized in that $R^1R^2R^3N^+$ represents a cyclic ammonium aromatic group selected from 1-pyridinium, 1-imidazolium or 3-thiazolium.

7. A process according to claim 3, characterized in that $R^1R^2R^3N^+$ is a non aromatic cyclic ammonium group selected from 1-pyrrolidinium, 1-piperidinium, 4-morpholinium, 1-methyl-1-pyrrolidinium, 1-methyl-1-piperidinium or 4-methyl-4-morpholinium.

**Patentansprüche**
**Patentansprüche für folgende Vertragsstaaten : AT, BE, CH, DE, DK, FR, GB, IT, LI, LU, NL, SE**

1. Verbindungen nach Formel (I)

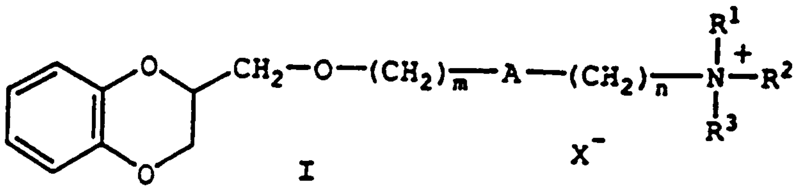

I

wobei $R^1$, $R^2$ und $R^3$, die gleich oder unterschiedlich sein können, Wasserstoff, geradkettiges oder verzweigtes $C_1$-$C_6$ Alkyl, Aryl oder Aralkyl, wobei der Alkylteil geradkettiges oder verzweigtes $C_1$-$C_6$ Alkyl ist, sind;
oder $R^1R^2R^3N^+$ eine aromatische cyclische Ammoniumgruppe ist, ausgewählt aus 1-Pyridinium, 1-Chinolinium, 2-Isochinolinium, 1-Imidazolium, 1-Pyrazolium, 3-Thiazolium, 3-Oxazolium, 1-Benzimidazolium, 3-Benzothiazolium oder 3-Benzoxazolium mit 1 bis 3 Substituenten an ihrem Ring, ausgewählt aus geradkettigem oder verzweigtem $C_1$-$C_6$ Alkyl, Halogen, Hydroxyalkyl, worin der Alkylteil geradkettiges oder verzweigtes $C_1$-$C_6$ Alkyl ist, Amino, Aminoalkyl, worin der Alkylteil geradkettiges oder verzweigtes $C_1$-$C_6$ Alkyl ist, oder Carboxy;
oder $R^1R^2R^3N^+$ eine nicht-aromatische cyclische Ammoniumgruppe ist, wobei zwei der Gruppen ($R^1$, $R^2$ oder $R^3$) zusammen mit dem Stickstoffatom einen Ring bilden, der ausgewählt ist aus 1-Pyrrolidin, 1-Piperidin, 4-Morpholin, 1-Piperazin oder 1-Perhydroazepin und die restliche Gruppe Wasserstoff oder geradkettiges oder verzweigtes $C_1$-$C_6$ Alkyl ist;
m und n, die gleich oder unterschiedlich sein können, eine ganze Zahl von 2 bis 12 sind;
A entweder eine kovalente Einfachbindung oder ein Sauerstoffatom ist;

$X^-$ ein pharmazeutisch brauchbares Anion ist,
Stereoisomere der Verbindungen (I) und Mischungen davon und pharmazeutisch brauchbare Salze davon.

**2.** Verbindungen wie in Anspruch 1 beansprucht, wobei m + n = 7-22.

**3.** Verbindungen nach Anspruch 1 oder 2, wobei $R^1$, $R^2$ und $R^3$ Wasserstoff, geradkettiges oder verzweigtes $C_1$-$C_6$ Alkyl, Aryl oder Aralkyl, worin der Alkylteil geradkettiges oder verzweigtes $C_1$-$C_6$ Alkyl ist, ist.

**4.** Verbindungen nach Anspruch 1 oder 2, wobei $R^1R^2R^3N^+$ eine aromatische cyclische Ammoniumgruppe ist, ausgewählt aus 1-Pyridinium, 1-Imidazolium oder 3-Thiazolium.

**5.** Verbindungen nach Anspruch 1 oder 2, wobei $R^1R^2R^3N^+$ eine nichtaromatische cyclische Ammoniumgruppe ist, ausgewählt aus 1-Pyrrolidinium, 1-Piperidinium, 4-Morpholinium, 1-Methyl-1-pyrrolinium, 1-Methyl-1-piperidinium oder 4-Methyl-4-morpholinium.

**6.** Verbindungen nach einem der Ansprüche 1-5, ausgewählt aus:
N-[10-[[(1,4-Benzodioxan-2-yl)methyl]oxy]decyl]-N,N,N-trimethylammonium,
N-[16-[[(1,4-Benzodioxan-2-yl)methyl]oxy]hexadecyl]-N,N,N-trimethylammonium,
N-[6-[[10-[[(1,4-Benzodioxan-2-yl)methyl]oxy]decyl]oxy]hexyl]-N,N,N-trimethylammonium,
N-[10-[[10-[[(1,4-Benzodioxan-2-yl)methyl]oxy]decyl]oxy]decyl]-N,N,N-trimethylammonium,
N-[10-[[(1,4-Benzodioxan-2-yl)methyl]oxy]decyl]-N,N,N-triethylammonium,
N-[16-[[(1,4-Benzodioxan-2-yl)methyl]oxy]hexadecyl]-N,N,N-triethylammonium,
N-[10-[[(1,4-Benzodioxan-2-yl)methyl]oxy]decyl]-N-methylpiperidinium,
N-[16-[[(1,4-Benzodioxan-2-yl)methyl]oxy]hexadecyl]-N-methylpiperidinium,
N-[6-[[10-[[(1,4-Benzodioxan-2-yl)methyl]oxy]decyl]oxy]hexyl]-N-methylpiperidinium,
N-[10-[[10-[[(1,4-Benzodioxan-2-yl)methyl]oxy]decyl]oxy]decyl]-N-methylpiperidinium,
N-[10-[[(1,4-Benzodioxan-2-yl)methyl]oxy]decyl]-N-methylmorpholinium,
N-[10-[[(1,4-Benzodioxan-2-yl)methyl]oxy]decyl]piperidinium,
N-[16-[[(1,4-Benzodioxan-2-yl)methyl]oxy]hexadecyl]piperidinium,
N-[10-[[6-[[(1,4-Benzodioxan-2-yl)methyl]oxy]decyl]oxy]hexyl]piperidinium,
N-[10-[[10-[[(1,4-Benzodioxan-2-yl)methyl]oxy]decyl]oxy]decyl]piperidinium,
N-[10-[[(1,4-Benzodioxan-2-yl)methyl]oxy]decyl]morpholinium,
(2S)-N-[10-[[(1,4-Benzodioxan-2-yl)methyl]oxy]decyl]piperidinium,
(2R)-N-[10-[[(1,4-Benzodioxan-2-yl)methyl]oxy]decyl]piperidinium,
wobei $X^-$ wie in Anspruch 1 definiert ist.

**7.** Verfahren zur Herstellung der Verbindungen, die in den Ansprüchen 1 bis 6 beansprucht werden, wobei in dem Prozeß eine Verbindung der Formel (II)

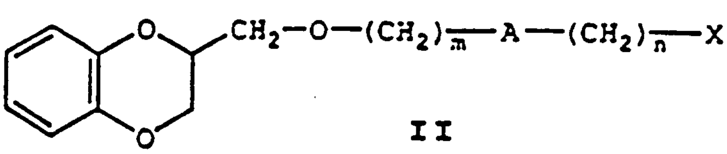

II

wobei X eine geeignete Abgangsgruppe ist und A, m und n die vorgenannte Bedeutung haben, mit einer Stickstoffbase der Formel $NR^1R^2R^3$, wobei $R^1$, $R^2$ und $R^3$ die vorgenannte Bedeutung haben, umgesetzt wird.

**8.** Verfahren nach Anspruch 7, wobei in dem Verfahren die Umsetzung in der Stickstoffbase $NR^1R^2R^3$ selbst als Lösungsmittel durchgeführt wird.

**9.** Pharmazeutische Zusammensetzung als wirksamen Bestandteil enthaltend eine Verbindung der Ansprüche 1 - 6 in Beimischung mit einem geeigneten Träger.

**Patentansprüche für folgende Vertragsstaaten : ES, GR**

1. Verfahren zur Herstellung von Verbindungen der Formel (I) und pharmazeutisch brauchbaren Salzen davon

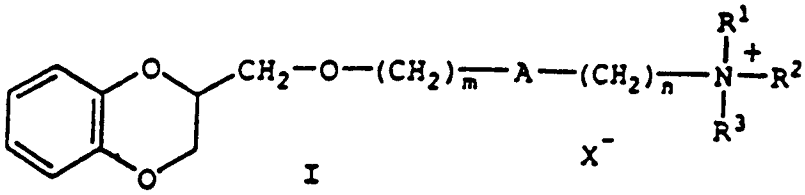

I

wobei $R^1$, $R^2$ und $R^3$, die gleich oder unterschiedlich sein können, Wasserstoff, geradkettiges oder verzweigtes $C_1$-$C_6$ Alkyl, Aryl oder Aralkyl, wobei der Alkylteil geradkettiges oder verzweigtes $C_1$-$C_6$ Alkyl ist, sind;
oder $R^1R^2R^3N^+$ eine aromatische cyclische Ammoniumgruppe ist, ausgewählt aus 1-Pyridinium, 1-Chinolinium, 2-Isochinolinium, 1-Imidazolium, 1-Pyrazolium, 3-Thiazolium, 3-Oxazolium, 1-Benzimidazolium, 3-Benzothiazolium oder 3-Benzoxazolium mit 1 bis 3 Substituenten an ihrem Ring, ausgewählt aus geradkettigem oder verzweigtem $C_1$-$C_6$ Alkyl, Halogen, Hydroxyalkyl, worin der Alkylteil geradkettiges oder verzweigtes $C_1$-$C_6$ Alkyl ist, Amino, Aminoalkyl, wobei der Alkylteil geradkettiges oder verzweigtes $C_1$-$C_6$ Alkyl ist, oder Carboxy;
oder $R^1R^2R^3N^+$ eine nicht-aromatische cyclische Ammoniumgruppe ist, wobei zwei der Gruppen ($R^1$, $R^2$ oder $R^3$) zusammen mit dem Stickstoffatom einen Ring bilden, der ausgewählt ist aus 1-Pyrrolidin, 1-Piperidin, 4-Morpholin, 1-Piperazin oder 1-Perhydroazepin und die restliche Gruppe Wasserstoff oder geradkettiges oder verzweigtes $C_1$-$C_6$ Alkyl ist;
m und n, die gleich oder unterschiedlich sein können, eine ganze Zahl von 2 bis 12 sind;
A entweder eine kovalente Einfachbindung oder ein Sauerstoffatom ist;
$X^-$ ein pharmazeutisch brauchbares Anion ist,
dadurch gekennzeichnet, daß eine Verbindung der Formel (II),

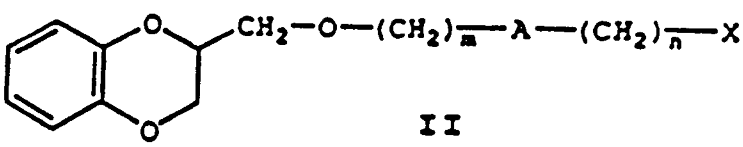

II

wobei X eine geeignete Abgangsgruppe ist, mit einer Stickstoffbase $R^1R^2R^3N$, wobei $R^1$, $R^2$ und $R^3$ die vorher definierte Bedeutung haben, umgesetzt wird.

2. Verfahren nach Anspruch 1, dadurch gekennzeichnet, daß, wenn eine der Gruppen $R^1$, $R^2$ und $R^3$ Wasserstoff ist, die Verbindung I wahlweise einer zweiten Umsetzung mit einer Verbindung der Formel $R^1X$, worin $R^1$ geradkettiges oder verzweigtes $C_1$-$C_6$ Alkyl oder eine Aralkylgruppe, worin der Alkylteil geradkettiges oder verzweigtes $C_1$-$C_6$ Alkyl ist und X eine der oben genannten Gruppen darstellt, unterzogen wird unter Bildung einer Verbindung I, wobei $R_1$ verschieden von Wasserstoff ist.

3. Verfahren nach Anspruch 1, dadurch gekennzeichnet, daß die Summe der Werte m und n von 7 bis 22 ist.

4. Verfahren nach Anspruch 3, dadurch gekennzeichnet, daß $R^1$, $R^2$ und $R^3$ Wasserstoff, $C_1$-$C_6$ Alkyl, Aryl oder Aralkylgruppen, wobei der Alkylteil $C_1$-$C_6$ Alkyl ist, sind.

5. Verfahren nach Anspruch 4, dadurch gekennzeichnet, daß $R^1$, $R^2$ und $R^3$ Methyl- oder Ethylgruppen sind.

6. Verfahren nach Anspruch 3, dadurch gekennzeichnet, daß $R^1R^2R^3N^+$ eine cyclische aromatische Ammoniumgruppe darstellt, ausgewählt aus 1-Pyridinium, 1-Imidazolium oder 3-Thiazolium.

7. Verfahren nach Anspruch 3, dadurch gekennzeichnet, daß $R^1R^2R^3N^+$ eine nicht-aromatische cyclische Ammoniumgruppe ist, ausgewählt aus 1-Pyrrolidinium, 1-Piperidinium, 4-Morpholinium, 1-Methyl-1-pyrrolidinium, 1-Methyl-1-piperidinium oder 4-Methyl-4-morpholinium.

## Revendications

**Revendications pour les Etats contractants suivants : AT, BE, CH, DE, DK, FR, GB, IT, LI, LU, NL, SE**

1. Composes de formule (I)

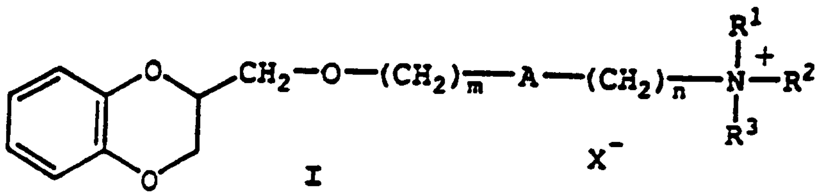

I

dans lesquels $R^1$, $R^2$ et $R^3$, qui peuvent être identiques ou différents, sont des atomes d'hydrogène, des groupes alkyles en $C_1$-$C_6$ linéaires ou ramifiés, aryles ou aralkyles dans lesquels la portion alkyle est un groupe alkyle en $C_1$-$C_6$ linéaire ou ramifié :
ou $R^1R^2R3N^+$ est un groupe ammonium aromatique cyclique, choisi parmi les groupes 1-pyridinium, 1-quinoléinium, 2-isoquinoléinium, 1-imidazolium, 1-pyrazolium, 3-thiazolium, 3-oxazolium, 1-benzimidazolium, 3-benzothiazolium ou 3-benzoxazolium, portant sur son cycle 1 à 3 substituants, choisis parmi des groupes alkyles en $C_1$-$C_6$ linéaire ou ramifie, des atomes d'halogène, des groupes hydroxyalkyles dans lesquels la portion alkyle est un groupe alkyle en $C^1$-$C^6$ linéaire ou ramifié, des groupes amino, des groupes aminoalkyles dans lesquels la portion alkyle est un groupe alkyle en $C_1$-$C_6$ linéaire ou ramifié, ou des groupes carboxy;
ou $R^1R^2R^3N^+$ est un groupe ammonium non aromatique cyclique, dans lequel deux des groupes ($R^1$, $R^2$, ou $R^3$), conjointement avec l'atome d'azote, forment un cycle choisi parmi les 1-pyrrolidine, 1-pipérédine, 4-morpholine, 1-pipérazine ou 1-perhydroazépine et le groupe restant est un atome d'hydrogène ou un groupe alkyle en $C_1$-$C_6$ linéaire ou ramifié ;
m et n, qui peuvent être identiques ou différents, sont des nombres entiers compris entre 2 et 12 ;
A est une liaison covalente simple ou un atome d'oxygène;
$X^-$ est un anion acceptable du point de vue pharmaceutique;
les stéréoisomères des composés (I) et les mélanges de ceux-ci, et les sels acceptables du point de vue pharmaceutique de ceux-ci.

2. Composés tels que revendiqués dans la revendication 1, dans lesquels $m + n = 7$ à 22.

3. Composés selon les revendications 1 ou 2, dans lesquels $R^1$, $R^2$ et $R^3$, sont des atomes d'hydrogène, des groupes alkyles en $C_1$-$C_6$ linéaires ou ramifiés, aryles ou aralkyles, dans lesquels la portion alkyle est un groupe alkyle en $C_1$-$C_6$ linéaire ou ramifié.

4. Composés selon les revendications 1 ou 2, dans lesquels $R^1R^2R^3N^+$ est un groupe ammonium aromatique cyclique, choisi parmi les groupes 1-pyridinium, 1-imidazolium, ou 3-thiazolium.

5. Composés selon les revendications 1 ou 2, dans lesquels $R^1R^2R^3N^+$ est un groupe ammonium non aromatique cyclique choisi parmi les groupes 1-pyrrolidinium, 1-pipéridinium, 4-morpholinium, 1-méthyl-1-pyrrolinium, 1-méthyl-1-pipéridinium ou 4-méthyl-4-morpholinium.

6. Composés selon l'une des revendications 1 à 5, choisis parmi :
   le N-[10-[[(1,4-benzodioxan-2-yl)méthyl]oxy]décyl]-N,N,N-triméthylammonium,
   le N-[16-[[(1,4-benzodioxan-2-yl)méthyl]oxy]hexadécyl]-N,N,N-triméthylammonium,
   le N-[6-[[10-[[(1,4-benzodioxan-2-yl)méthyl]oxy)décyl]oxy]hexyl]-N,N,N-triméthylammonium,
   le N-[10-[[10-[[(1,4-benzodioxan-2-yl)méthyl]oxy)décyl]oxy]hexyl]-N,N,N-triméthylammonium,
   le N-[10-[[(1,4-benzodioxan-2-yl)méthyl]oxy)décyl]-N,N,N-triméthylammonium,
   le N-[16-[[(1,4-benzodioxan-2-yl)méthyl]oxy)décyl]oxy]hexadécyl]-N,N,N-triéthylammonium,
   le N-[10-[[10-[[(1,4-benzodioxan-2-yl)méthyl]oxy]décyl]-N-méthylpipéridinium,

le N-[16-[[(1,4-benzodioxan-2-yl)méthyl]oxy]hexadécyl]-N-méthylpipéridinium,
le N-[6-[[10-[[(1,4-benzodioxan-2-yl)méthyl]oxy]décy]oxy]hexyl]-N-méthylpipéridinium,
le N-[10-[[10-[[(1,4-benzodioxan-2-yl)méthyl]oxy]décyl]oxy]décyl]-N-méthylpipéridinium,
le N-[10-[[(1,4-benzodioxan-2-yl)méthyl]oxy]décyl]-N-méthylmorpholinium,
le N-[10-[[(1,4-benzodioxan-2-yl)méthyl]oxy]décyl]-pipéridinium,
le N-[16-[[(1,4-benzodioxan-2-yl)méthyl]oxy]hexadécyl]-pipéridinium,
le N-[10-[[6-[[(1,4-benzodioxan-2-yl)méthyl]oxy]décyl]oxy]hexyl]-pipéridinium,
le N-[10-[[10-[[(1,4-benzodioxan-2-yl)méthyl]oxy]décyl]oxy]décyl]-pipéridinium,
le N-[10-[[(1,4-benzodioxan-2-yl)méthyl]oxy]décyl]-morpholinium,
le (2S)-N-[10-[[(1,4-benzodioxan-2-yl)méthyl]oxy]décyl]-pipéridinium,
le (2R)-N-[10-[[(1,4-benzodioxan-2-yl)méthyl]oxy]décyl]-pipéridinium,
dans lequel $X^-$ est tel que défini dans la revendication 1.

7. Procédé de préparation des composés revendiqués dans les revendications 1 à 6, dans lequel on fait réagir un composé de formule (II)

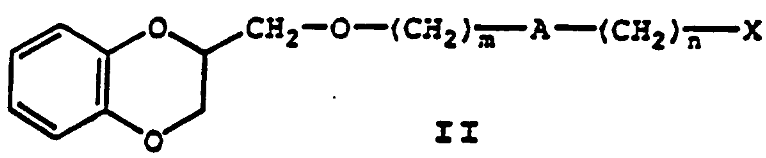

II

dans lequel X est un groupe labile approprié, et A, m et n ont les significations susmentionnées, avec une base azotée de formule $NR^1R^2R^3$ dans laquelle $R^1$, $R^2$, et $R^3$ ont les significations susmentionnées.

8. Procédé selon la revendication 7, dans lequel la réaction est mise en oeuvre dans la base azotée $NR^1R^2R^3$ elle-même comme solvant.

9. Compositions pharmaceutiques contenant comme principe actif un composé des revendications 1 à 6 en mélange avec un véhicule approprié.

**Revendications pour les Etats contractants suivants : ES, GR**

1. Un procédé de préparation de composés de formule (1) et de ses sels pharmaceutiquement acceptables

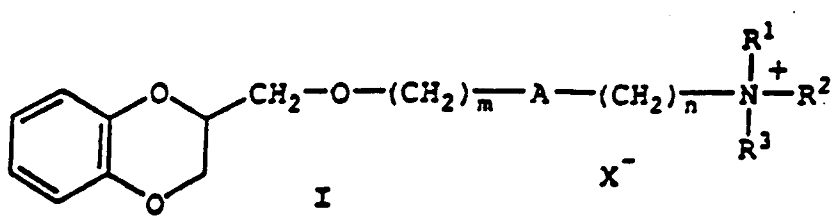

I

dans lesquels $R^1$, $R^2$ et $R^3$, qui peuvent être identiques ou différents, sont des atomes d'hydrogène, des groupes alkyles en $C_1$-$C_6$ linéaires ou ramifiés, aryles ou aralkyles dans lesquels la portion alkyle est un groupe alkyle en $C_1$-$C_6$ linéaire ou ramifié :
ou $R^1R^2R^3N^+$ est un groupe ammonium aromatique cyclique, choisi parmi les groupes 1-pyridinium, 1-quinoléinium, 2-isoquinoléinium, 1-imidazolium, 1-pyrazolium, 3-thiazolium, 3-oxazolium, 1-benzimidazolium, 3-benzothiazolium ou 3-benzoxazolium, portant sur son cycle 1 à 3 substituants, choisis parmi des groupes alkyles en $C_1$-$C_6$ linéaire ou ramifié, des atomes d'halogène, des groupes hydroxyalkyles dans lesquels la portion alkyle est un groupe alkyle en C1-$C^6$ linéaire ou ramifié, des groupes amino, des groupes aminoalkyles dans lesquels la portion alkyle est un groupe alkyle en $C_1$-$C_6$ linéaire ou ramifié, ou des groupes carboxy;
ou $R^1R^2R^3N^+$ est un groupe ammonium non aromatique cyclique, dans lequel deux des groupes ($R^1$, $R^2$, ou $R^3$), conjointement avec l'atome d'azote, forment un cycle choisi parmi les 1-pyrrolidine, 1-pipérédine, 4-morpholine, 1-pipérazine ou 1-perhydroazépine et le groupe restant est un atome

d'hydrogène ou un groupe alkyle en $C_1$-$C_6$ linéaire ou ramifié ;
m et n, qui peuvent être identiques ou différents, sont des nombres entiers compris entre 2 et 12 ;
A est une liaison covalente simple ou un atome d'oxygène;
$X^-$ est un anion acceptable du point de vue pharmaceutique;
caractérisé en ce qu'on fait réagir un composé de formule (II)

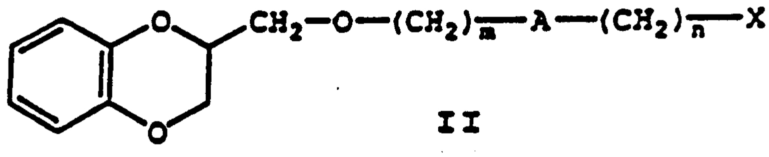

II

dans lequel X est un groupe labile approprié, avec une base azotée de formule $NR^1R^2R^3$ dans laquelle $R^1$, $R^2$, et $R^3$ ont les significations susmentionnées.

2. Un procédé selon la revendication 1, caractérisé en ce que, lorsqu'un des groupes $R^1$, $R^2$ et $R^3$ est l'hydrogène, ledit composé (I) est facultativement soumis à une deuxième réaction avec un composé $R^1X$, où $R^1$ est un groupe alkyle linéaire ou ramifié en $C_1$-$C_6$ ou un groupe aralkyle ayant une portion alkyle linéaire ou ramifiée en $C_1$-$C_6$, et X représente les groupes sus-mentionnés, pour obtenir un composé (I) dans lequel $R^1$ est différent de l'hydrogène.

3. Un procédé selon la revendication 1, caractérisé en ce que $m + n = 7$ à 22.

4. Un procédé selon la revendication 3, caractérisé en ce que $R^1$, $R^2$ et $R^3$, sont des atomes d'hydrogène, des groupes alkyles en $C_1$-$C_6$ linéaires ou ramifiés, aryles ou aralkyles, dans lesquels la portion alkyle est un groupe alkyle en $C_1$-$C_6$ linéaire ou ramifié.

5. Un procédé selon la revendication 4, caractérisé en ce que $R^1$, $R^2$ et $R^3$ sont des groupes méthyle ou éthyle.

6. Un procédé selon la revendication 3, caractérisé en ce que $R^1R^2R^3N^+$ est un groupe ammonium aromatique cyclique, choisi parmi les groupes 1-pyridinium, 1-imidazolium, ou 3-thiazolium.

7. Un procédé selon la revendicatin 3, caractérisé en ce que $R^1R^2R^3N^+$ est un groupe ammonium non aromatique cyclique choisi parmi les groupes 1-pyrrolidinium, 1-pipéridinium, 4-morpholinium, 1-méthyl-1-pyrrolinium, 1-méthyl-1-pipéridinium ou 4-méthyl-4-morpholinium.